# EUROPEAN PATENT APPLICATION

(11) **EP 4 141 017 A1**
(43) Date of publication of application: **01.03.2023**
(21) Application number: 21792412.5
(22) Date of filing: 23.04.2021
(51) Int. Cl.: C07K 5/04, A61K 38/05, A61K 47/62, A61K 47/68, A61P 35/00, C07K 7/04, C07K 19/00

(54) **DUOCARMYCIN DERIVATIVE AND USE THEREOF**

(30) Priority: 24.04.2020 JP 2020077058
(71) Applicant: The University of Tokyo, Bunkyo-ku, Tokyo 113-8654 (JP); Savid Therapeutics Inc., Tokyo 168-0064 (JP); Tohoku University, Sendai-shi, Miyagi 980-8577 (JP)
(72) Inventor: KANAI, Motomu, Tokyo 113-8654 (JP); YAMATSUGU, Kenzo, Tokyo 113-8654 (JP); TATSUMI, Toshifumi, Tokyo 113-8654 (JP); KODAMA, Tatsuhiko, Tokyo 113-8654 (JP); SUGIYAMA, Akira, Tokyo 113-8654 (JP); TSUKAGOSHI, Masanobu, Tokyo 168-0064 (JP); TOKUYAMA, Hidetoshi, Sendai-shi, Miyagi 980-8577 (JP); SAKATA, Juri, Sendai-shi, Miyagi 980-8577 (JP)
(74) Representative: Godemeyer Blum Lenze Patentanwälte Partnerschaft mbB - werkpatent
(86) International application number: PCT/JP2021/016484
(87) International publication number: WO 2021/215534

(57) **Abstract**

It is an object of the present invention to provide a conjugate of a duocarmycin derivative and a biotin-modified dimer, which is useful for pretargeting methods. According to the present invention, a compound represented by the following formula (1) or formula (2) is provided: 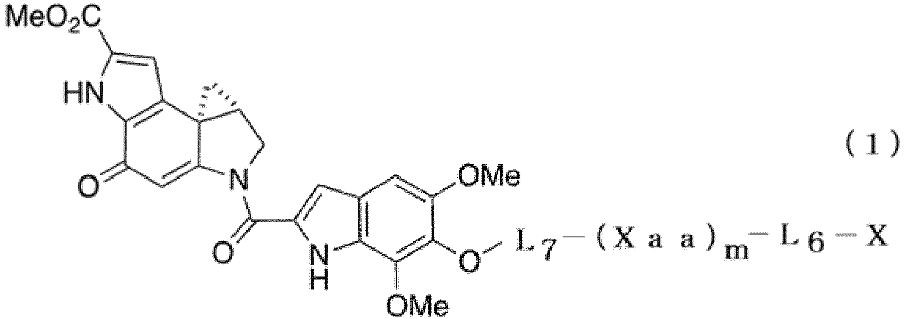 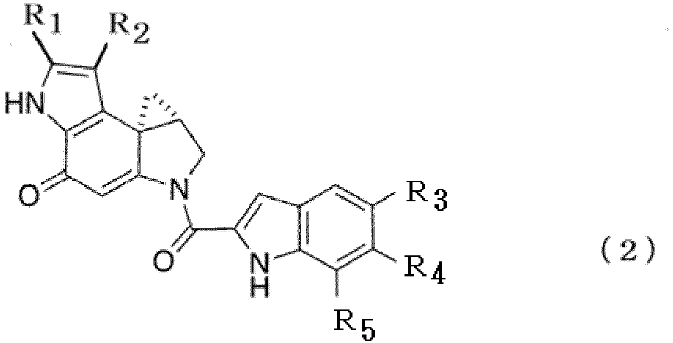 wherein R₁ and R₂ each independently represent a hydrogen atom, a lower alkyl group, or a lower alkoxycarbonyl group; one of R₃, R₄, and R₅ represents -O-L₇-(Xaa)ₘ-L₆-N₃, and the remaining two each independently represent a hydrogen atom, a lower alkyl group, or a lower alkoxycarbonyl group; X represents a reactive group; L₆ and L₇ each independently represent a divalent linking group; Xaa represents an amino acid residue; m represents an integer of 2 to 10; and Me represents a methyl group.

## Description

### Technical Field

The present invention relates to a conjugate of a duocarmycin derivative and a biotin-modified dimer, and the use thereof.

### Background Art

Avidin and biotin, or streptavidin and biotin have an extremely high affinity between them (Kd = 10⁻¹⁵ to 10⁻¹⁴ M). This is one of the extremely strong interactions between two biomolecules. Presently, the interaction between avidin/streptavidin and biotin has been widely applied in biochemistry, molecular biology, and medicine. A drug delivery method and a pretargeting method, in which high binding ability between avidin/streptavidin and biotin is combined with an antibody molecule, have been devised. A mutant streptavidin with a reduced affinity for natural biotin, and a biotin-modified dimer having a high affinity for the mutant streptavidin above with a low affinity for natural biotin, are reported in Patent Document 1.

Duocarmycin is a group of natural products isolated from Streptomyces sp., and various analogs have been isolated and/or synthesized from duocarmycin. These compounds having dienone cyclopropane as a common structure exhibit DNA alkylating action and strong antitumor activity. The dienone cyclopropane ring is generally poor in terms of electrophilicity. When duocarmycin binds to a minor group of double-stranded DNA, the conformation is fixed in a staggered form. This time, conjugation of the dienone with a lone-pair nitrogen atom becomes impossible, and the electrophilicity of the dienone portion is improved. As a result, alkylation of adenine bases of DNA progresses in the cyclopropane portion, and high anticancer action is exhibited.

Duocarmycins exhibiting strong antitumor activity are attractive as anticancer agents, and thus, duocarmycins have been studied for clinical application. However, since duocarmycins are problematic in terms of delayed hepatotoxicity after the use thereof, off-target problem caused by low molecular weight drugs, and the like, duocarmycins alone have not yet been approved as pharmaceutical products.

Meanwhile, Patent Document 2 discloses a toxin complex, in which a residue derived from a compound having an affinity for target cells is allowed to bind to a toxin residue via a spacer containing polyalkylene glycol and dipeptide. In addition, Non-Patent Document 1 discloses the synthesis of a novel duocarmycin derivative DU-257, and application of the duocarmycin derivative to an immunoconjugate, using a poly(ethylene glycol) dipeptidyl linker capable of tumor-specific activation.

### Prior Art Documents

### Patent Documents

Patent Document 1: International Publication WO2015/125820
Patent Document 2: International Publication WO96/35451

### Non-Patent Documents

Non-Patent Document 1: Bioorganic & Medicinal Chemistry 8 (2000) 2175-2184

### Summary of Invention

### Object to be Solved by the Invention

It is an object of the present invention to provide a conjugate of a duocarmycin derivative and a biotin-modified dimer, which is useful for pretargeting methods. Moreover, it is another object of the present invention to provide a therapeutic kit, in which a combination of the above-described conjugate of a duocarmycin derivative and a biotin-modified dimer, with a mutant streptavidin-molecular probe conjugate, is used.

### Means for Solving the Object

As a result of intensive studies directed towards achieving the above-described objects, the present inventor has found that cell growth can be effectively suppressed by synthesizing a novel conjugate of a duocarmycin derivative and a biotin-modified dimer, and then using this conjugate in combination with a mutant streptavidin-molecular probe conjugate, thereby completing the present invention.

Specifically, according to the present invention, the following inventions are provided.
<1> A compound represented by the following formula (1) or formula (2): 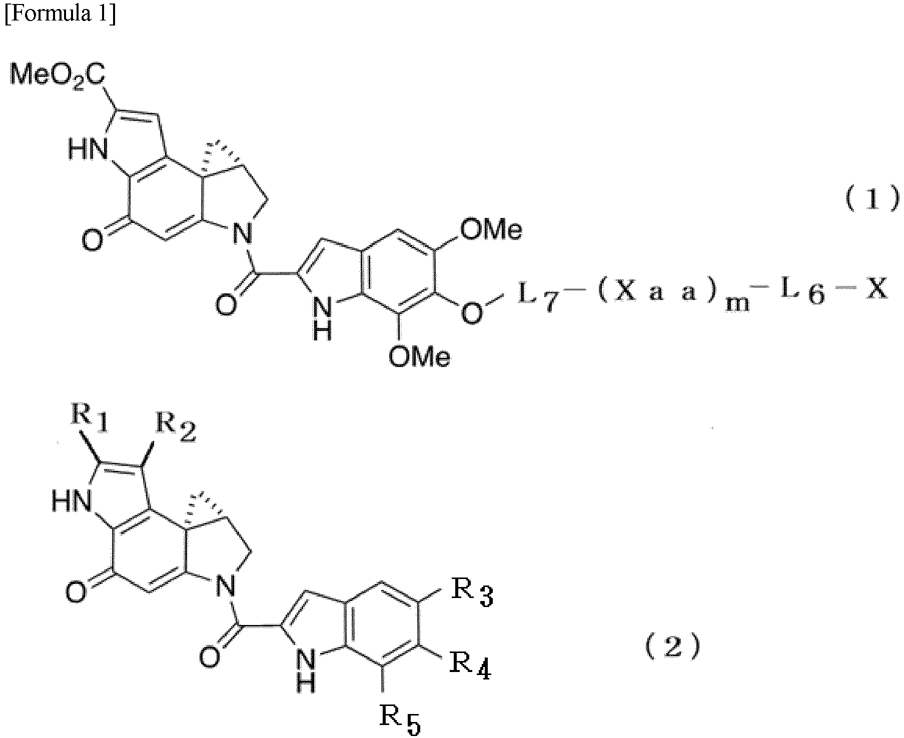 wherein R₁ and R₂ each independently represent a hydrogen atom, a lower alkyl group, or a lower alkoxycarbonyl group; one of R₃, R₄, and R₅ represents -O-L₇-(Xaa)ₘ-L₆-N₃, and the remaining two each independently represent a hydrogen atom, a lower alkyl group, or a lower alkoxycarbonyl group; X represents a reactive group; L₆ and L₇ each independently represent a divalent linking group; Xaa represents an amino acid residue; m represents an integer of 2 to 10; and Me represents a methyl group.
<2> The compound according to <1>, which is represented by the following formula (3A) or formula (3B): 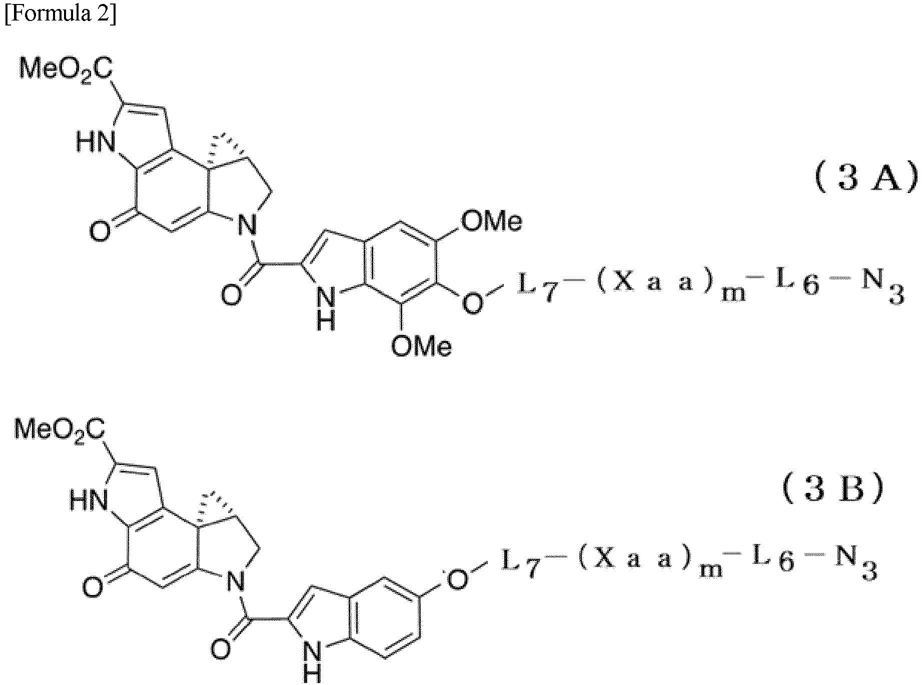 wherein the symbols are as defined in claim 1.
<3> The compound according to <1> or <2>, wherein -(Xaa)ₘ- is -Val-Ala-.
<4> The compound according to any one of <1> to <3>, wherein L₆ is -CO-(-O-CH₂)ₙ-(wherein n represents an integer of 1 to 10), and L₇ is -(CH₂)ₚ-NH- (wherein p represents an integer of 1 to 5).
<5> A compound represented by the following formula (4): 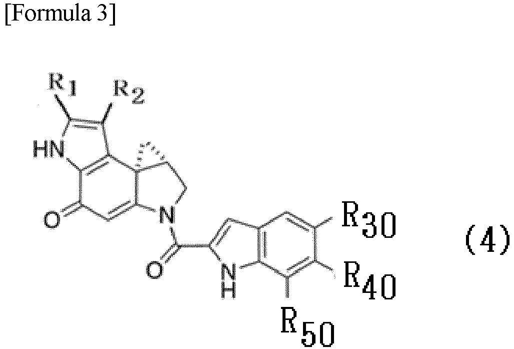 wherein
   R₁ and R₂ each independently represent a hydrogen atom, a lower alkyl group, or a lower alkoxycarbonyl group; and
   one of R₃₀, R₄₀ and R₅₀ represents the following: 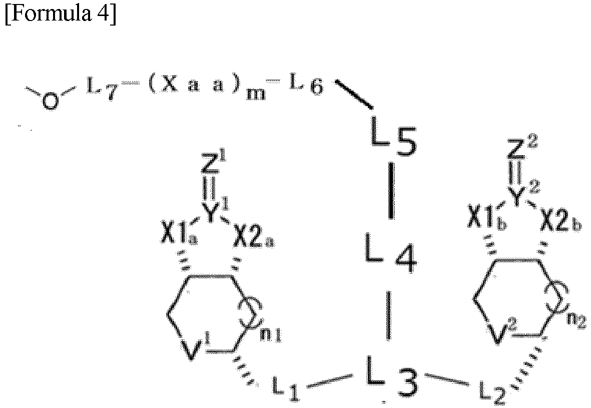
   and the remaining two each independently represent a hydrogen atom, a lower alkyl group, or a lower alkoxyl group, wherein
   X1a, X1b, X2a and X2b each independently represent O or NH;
   Y¹ and Y² each independently represent C or S;
   Z¹ and Z² each independently represent O, S or NH;
   V¹ and V² each independently represent S or S⁺-O⁻, and n1 and n2 each independently represent an integer of 0 or 1;
   L₁ and L₂ each independently represent a divalent linking group;
   L₃ represents a trivalent linking group;
   L₄, L₅, L₆ and L₇ each independently represent a divalent linking group; and
   Xaa represents an amino acid residue, and m represents an integer of 2 to 10.
<6> The compound according to <5>, wherein
   one of R₃₀, R₄₀ and is the following: 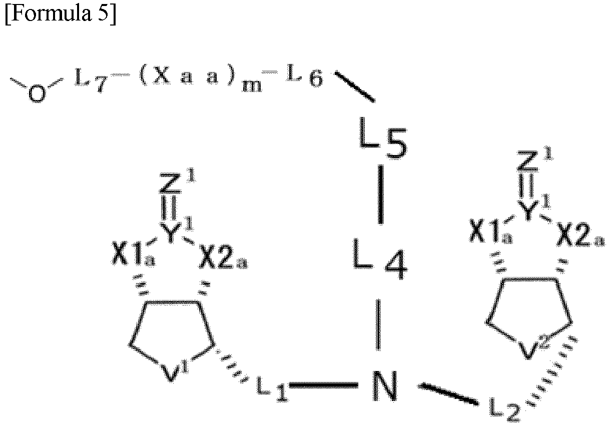 wherein individual symbols are as defined in claim 5.
<7> A compound represented by the following formula (4A): 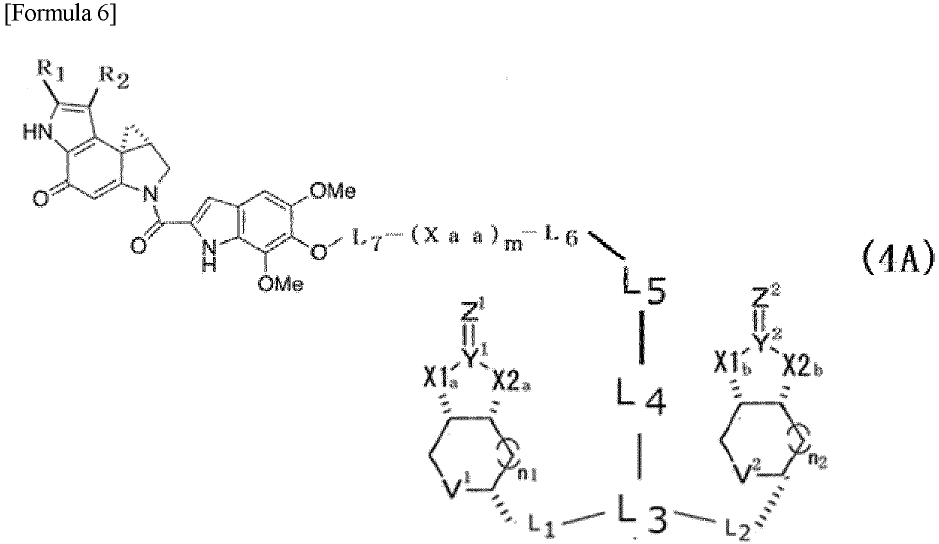 wherein
   X1a, X1b, X2a and X2b each independently represent O or NH;
   Y¹ and Y² each independently represent C or S;
   Z¹ and Z² each independently represent O, S or NH;
   V¹ and V² each independently represent S or S⁺-O⁻, and n1 and n2 each independently represent an integer of 0 or 1;
   L₁ and L₂ each independently represent a divalent linking group;
   L₃ represents a trivalent linking group;
   L₄, L₅, L₆ and L₇ each independently represent a divalent linking group;
   Xaa represents an amino acid residue, and m represents an integer of 2 to 10;
   Me represents a methyl group; and
   R₁ and R₂ each independently represent a hydrogen atom, a lower alkyl group, or a lower alkoxycarbonyl group.
<8> The compound according to <5>, which is represented by the following formula (5): 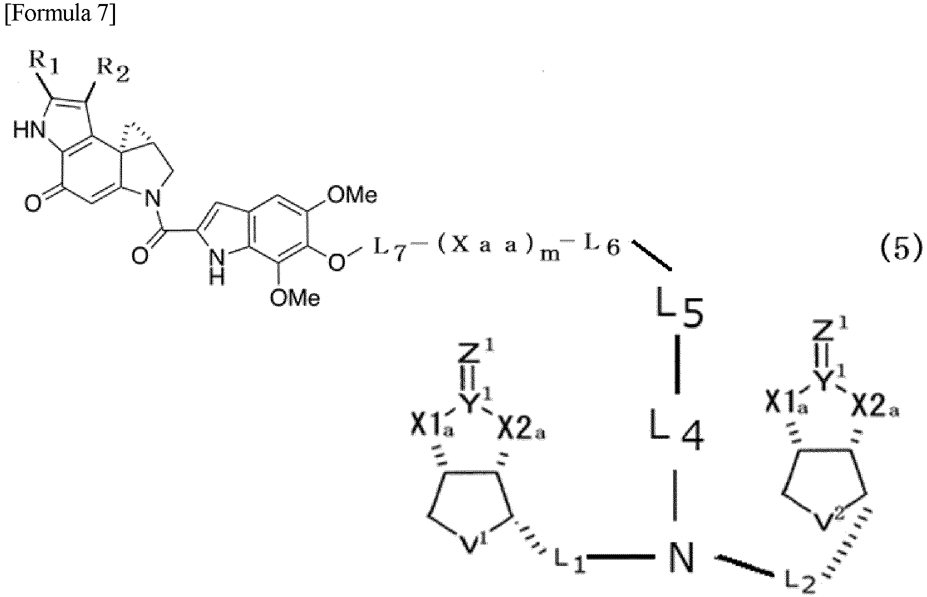 wherein individual symbols are as defined in claim 5.
<9> The compound according to any one of <5> to <8>, wherein X1a, X1b, X2a and X2b represent NH; Y¹ and Y² represent C; Z¹ and Z² represent NH; and V¹ and V² represent S.
<10> The compound according to any one of <5> to <9>, wherein L₁ and L₂ each independently represent a divalent group consisting of a combination of groups selected from -CONH-, -NHCO-, - COO-, -OCO-, -CO-, -O-, and an alkylene group containing 1 to 10 carbon atoms.
<11> The compound according to any one of <5> to <10>, wherein L₄ represents a group consisting of a combination of groups selected from -CONH-, -NHCO-, -COO-, -OCO-, -CO-, -O-, - NH-, and an alkylene group containing 1 to 10 carbon atoms.
<12> The compound according to any one of<5>to<11<, wherein L₅ represents a triazole group, -S-, -CONH-, -NHCO-, -COO-, -OCO-, -CO-, -O-, -NH-, an alkylene group containing 1 to 10 carbon atoms, or a group consisting of a combination of these groups.
<13> The following compound or a salt thereof: 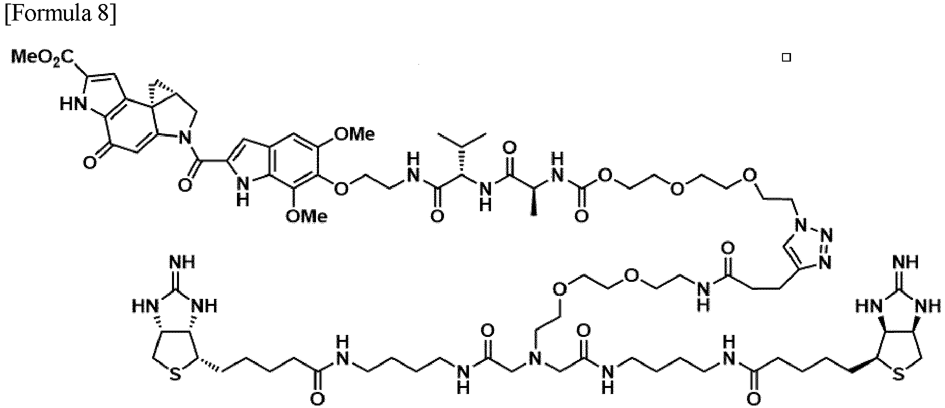
<14> A therapeutic kit comprising:
   (a) the compound according to any one of <5> to <13>; and
   (b) a conjugate of a mutant streptavidin comprising the amino acid sequence as set forth in SEQ ID NO: 1 (provided that a part of or the entire histidine tag at the C-terminus may be deleted) and a molecular probe.
<15> The therapeutic kit according to <14>, wherein the molecular probe is an anti-EREG antibody, an anti-CEA antibody, or an anti-HER2 antibody.

### Advantageous Effects of Invention

By using the conjugate of the present invention, the growth of cancer cells can be effectively suppressed.

### Brief Description of Drawings

[Fig. 1] Fig. 1 shows an outline of a domain structure.
[Fig. 2] Fig. 2 shows a CBB-stained SDS-PAGE electrophoretic pattern of CEA-V2122.
[Fig. 3] Fig. 3 shows the evaluation of the binding performance of CEA-V2122 with an antigen (CEACAM5).
[Fig. 4] Fig. 4 shows the evaluation of the binding performance of CEA-V2122 with a modified biotin.
[Fig. 5] Fig. 5 shows the stained cell images obtained using FITC-labeled CEA-V2122.
[Fig. 6] Fig. 6 shows the time-series data of stained MKN45 cell images obtained using FITC-labeled CEA-V2122.
[Fig. 7] Fig. 7 shows the structure of Compound 5.
[Fig. 8] Fig. 8 shows the measurement results of a cytotoxicity test, in which CEA-V2122 and an anticancer agent-labeled modified biotin.
[Fig. 9] Fig. 9 shows the structure of Compound X (Duocarmycin SA).
[Fig. 10] Fig. 10 shows the structure of Compound Y (Duocarmycin SA-Valin).
[Fig. 11] Fig. 11 shows the measurement results of a cytotoxicity test regarding Compound X (Duocarmycin SA) and Compound Y (Duocarmycin SA-Valin).

### Embodiments of Carrying out the Invention

Hereinafter, the present invention will be described in more detail.

### (1) Duocarmycin derivative

The duocarmycin derivative of the present invention is a compound formed by binding a linker to duocarmycin, and it is specifically a compound represented by the following formula (1) or formula (2) (for example, the following formula (2A) or formula (2B)):

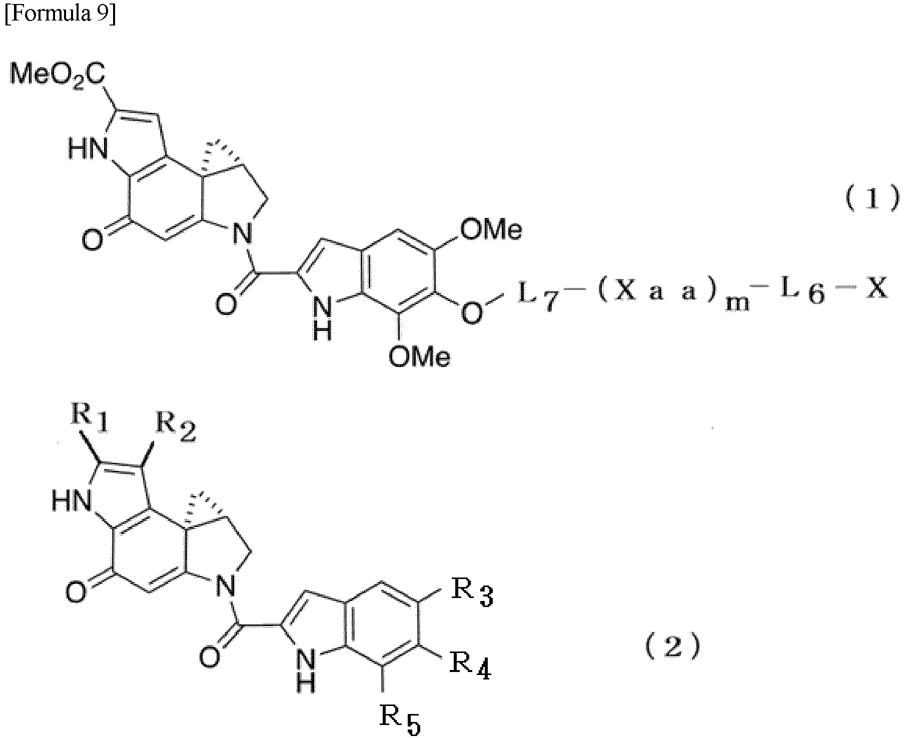

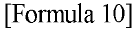

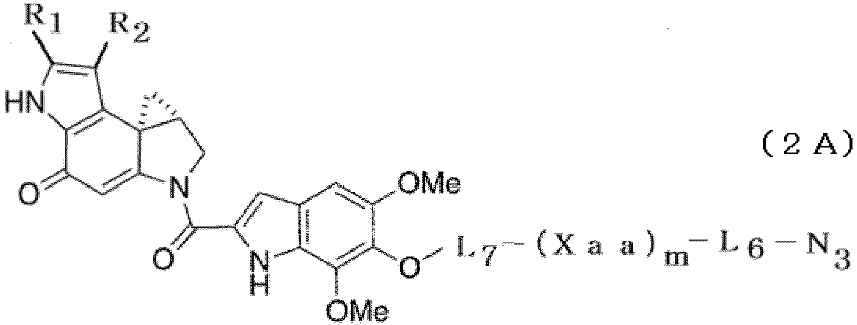

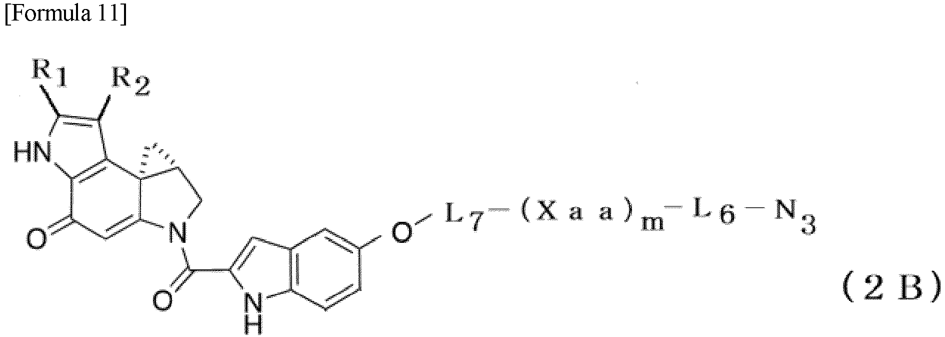

The compound of the present invention is more preferably a compound represented by the following formula (3A) or the following formula (3B):

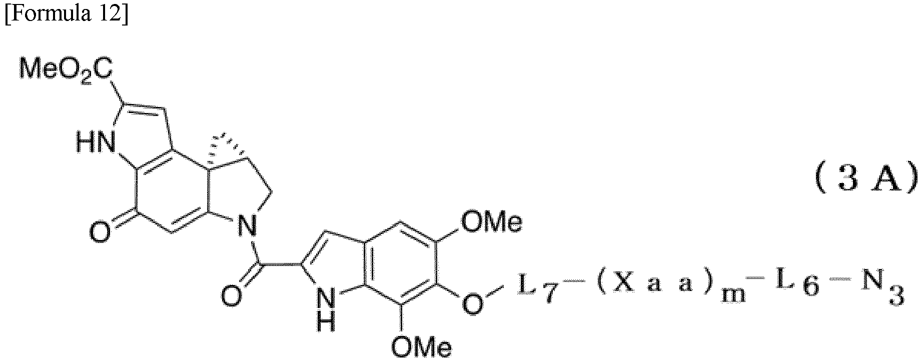

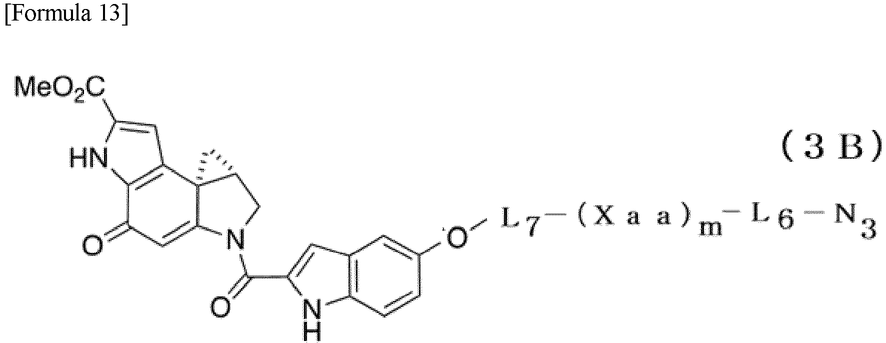

In the above formula (1), X represents a reactive group. Examples of the reactive group may include, but are not particularly limited to, -N₃, carbodiimide (for example, EDC), NHS ester, imide ester, maleimide, haloacetic acid (bromoacetic acid or iodoacetic acid), pyridyl disulfide, thiosulfone, vinyl sulfone, hydrazide, alkoxyamine, arylazide, isocyanate, alkyne, alkene, and tetrazine.

In the above formula (2), one of R₃, R₄, and R₅ represents -O-L₇-(Xaa)ₘ-L₆-N₃, and the remaining two each independently represent a hydrogen atom, a lower alkyl group (for example, an alkyl group containing 1 to 6 carbon atoms, and preferably an alkyl group containing 1 to 4 carbon atoms), or a lower alkoxyl group (for example, an alkoxyl group containing 1 to 6 carbon atoms, and preferably an alkoxyl group containing 1 to 4 carbon atoms).

In the above formula (1) to formula (3), L₆ and L₇ each independently represent a divalent linking group. The divalent linking group may be, for example, a group consisting of a combination of -CO-, -O-, -CH₂-, and -NH-. The divalent linking group may be more specifically a group consisting of a combination of -CO-, (-O-CH₂)ₙ- (wherein n represents an integer of 1 to 10), - (CH₂)ₚ (wherein p represents an integer of 1 to 5), and -NH-. One example of L₆ may be -CO-(-O-CH₂)ₙ- (wherein n represents an integer of 1 to 10). One example of L₇ may be -(CH₂)ₚ-NH-(wherein p represents an integer of 1 to 5).

In the above formula (1) to formula (3), Xaa represents an amino acid residue, and m represents an integer of 2 to 10, preferably an integer of 2 to 7, more preferably an integer of 2 to 5, and further preferably 2, 3 or 4.

The amino acid residue may be any one of an alanine residue, a cysteine residue, an aspartic acid residue, a glutamic acid residue, a phenylalanine residue, a glycine residue, a histidine residue, an isoleucine residue, a lysine residue, a leucine residue, a methionine residue, an asparagine residue, a proline residue, a glutamine residue, an arginine residue, a serine residue, a threonine residue, a valine residue, a tryptophan residue, a tyrosine residue, and a citrulline residue (Cit). The amino acid residues are preferably a valine residue and an alanine residue. Specific examples of - (Xaa)ₘ- may include - Val-Ala-, -Phe-Lys-, and -Val-Cit-, but the examples are not particularly limited thereto.

In the above formula (1) to formula (3), Me represents a methyl group.

In the above formula (2), R₁ and R₂ each independently represent a hydrogen atom, a lower alkyl group (for example, an alkyl group containing 1 to 6 carbon atoms, and preferably an alkyl group containing 1 to 4 carbon atoms), or a lower alkoxycarbonyl group (preferably an alkoxycarbonyl group containing 2 to 7 carbon atoms, and more preferably an alkoxycarbonyl group containing 2 to 5 carbon atoms).

The duocarmycin derivative of the present invention can be synthesized in accordance with the method described in Examples later.

### (2) Conjugate

The present invention relates to a conjugate of a duocarmycin derivative and a biotin-modified dimer, and the conjugate is a compound represented by the following formula (4) or a salt thereof. The conjugate of the present invention is preferably a compond represented by the following formula (4A) or a salt thereof, or a compound represented by the following formula (5) or a salt thereof 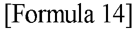 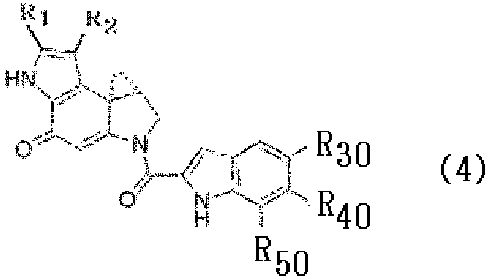 wherein
R₁ and R₂ each independently represent a hydrogen atom, a lower alkyl group, or a lower alkoxycarbonyl group; and
one of R₃₀, R₄₀ and R₅₀ represents the following: 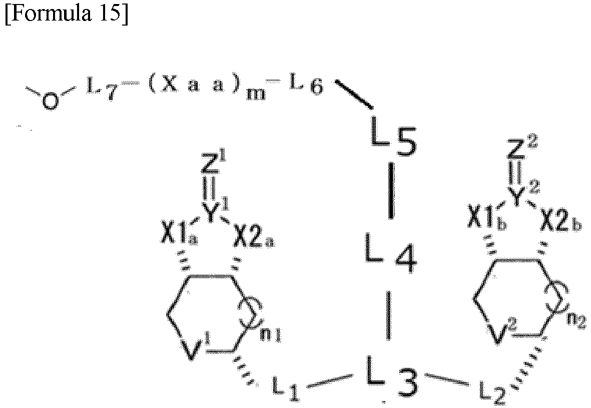
and the remaining two each independently represent a hydrogen atom, a lower alkyl group, or a lower alkoxyl group, wherein
X1a, X1b, X2a and X2b each independently represent O or NH;
Y¹ and Y² each independently represent C or S;
Z¹ and Z² each independently represent O, S or NH;
V¹ and V² each independently represent S or S⁺-O⁻, and n1 and n2 each independently represent an integer of 0 or 1;
L₁ and L₂ each independently represent a divalent linking group;
L₃ represents a trivalent linking group;
L₄, L₅, L₆ and L₇ each independently represent a divalent linking group; and
Xaa represents an amino acid residue, and m represents an integer of 2 to 10.

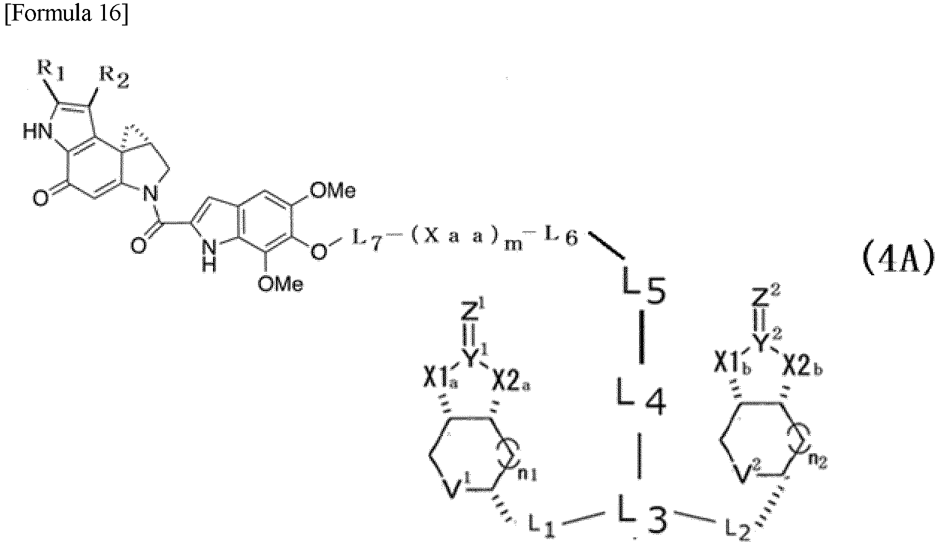

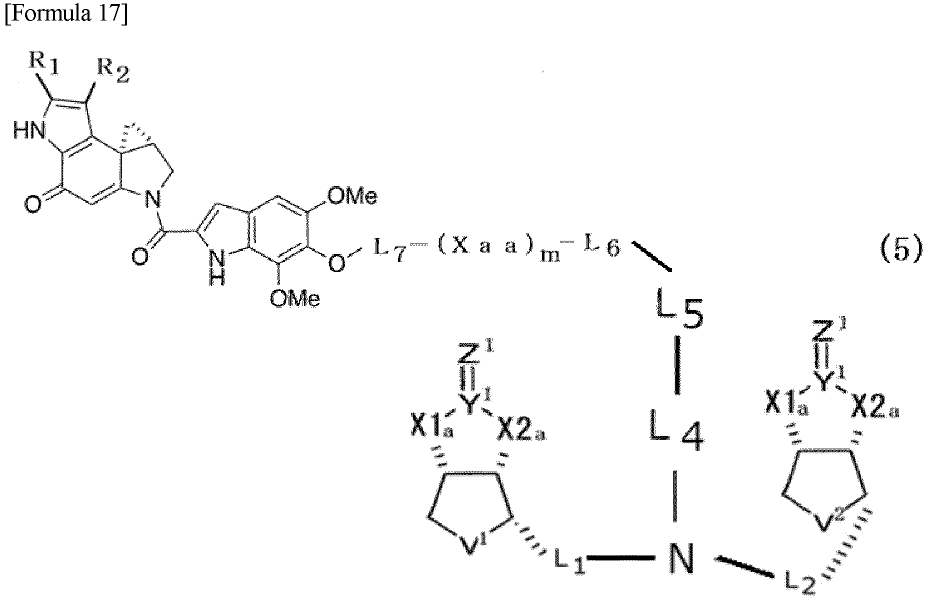

In the above formulae, one of R₃₀, R₄₀ and R₅₀ is preferably the following:

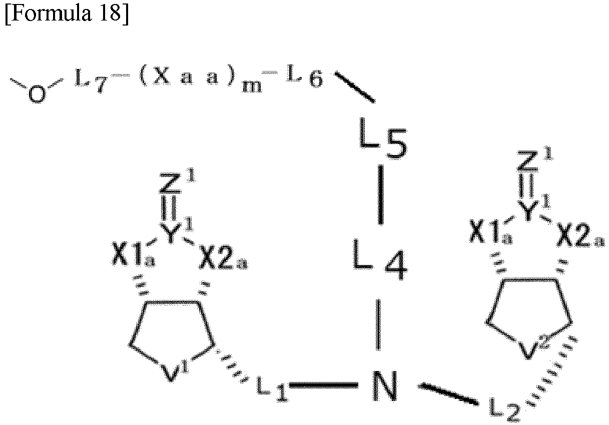

The biotin-modified dimer moiety is a compound represented by the following formula (11) or a salt thereof, and is preferably a compound represented by the following formula (12) or a salt thereof. As such a biotin-modified dimer, the compound described in International Publication WO2015/125820 can be used. 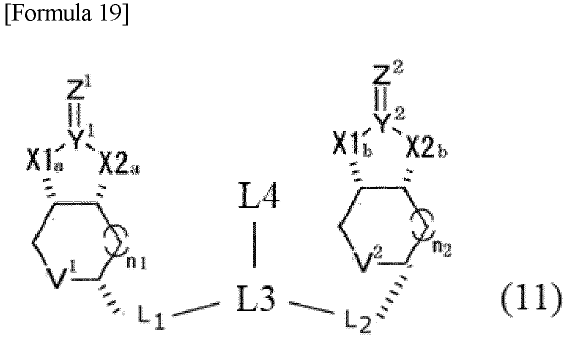 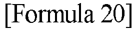 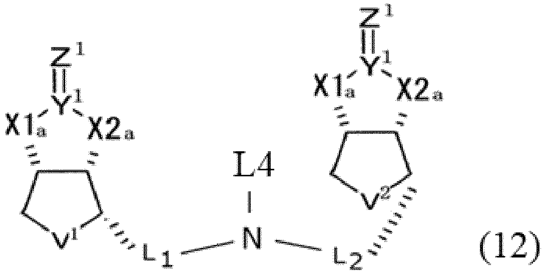 wherein, in the above formulae,
X1a, X1b, X2a and X2b each independently represent O or NH;
Y¹ and Y² each independently represent C or S;
Z¹ and Z² each independently represent O, S or NH;
V¹ and V² each independently represent S or S⁺-O⁻, and n1 and n2 each independently represent an integer of 0 or 1;
L₁ and L₂ each independently represent a divalent linking group;
L₃ represents a trivalent linking group; and
L₄ represents a divalent linking group.

In the above formulae, the portions represented by the following structures: 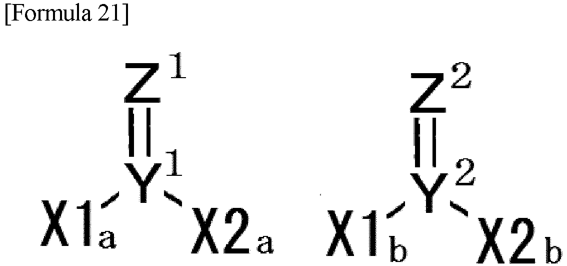 are preferably any one of the following portions, but are not limited thereto:

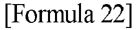

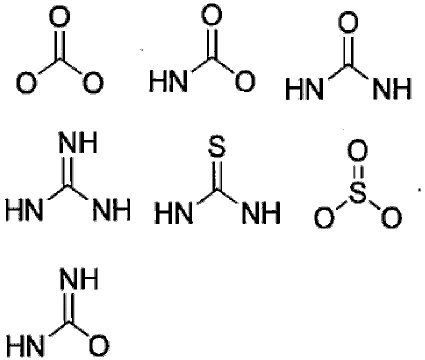

X1a, X1b, X2a and X2b preferably represent NH; Y¹ and Y² preferably represent C; Z¹ and Z² preferably represent NH; and V¹ and V² preferably represent S.

L₁ and L₂ each independently represent a divalent linking group consisting of a combination of groups selected from -CONH-, -NHCO-, -COO-, -OCO-, -CO-, -O-, and an alkylene group containing 1 to 10 carbon atoms.

Preferably, L₁ and L₂ each independently represent a divalent linking group consisting of a combination of groups selected from -CONH-, -NHCO-, -O-, and an alkylene group containing 1 to 10 carbon atoms.

Preferably, L₁ and L₂ each independently represent a divalent linking group consisting of a combination of groups selected from -CONH-, -NHCO-, and an alkylene group containing 1 to 10 carbon atoms.

L₃ represents a trivalent linking group, and is preferably the following: 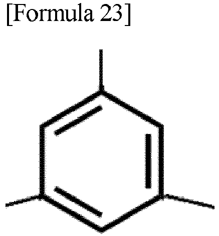 or, 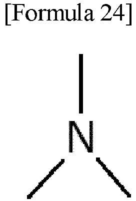 (which is a benzene-derived trivalent linking group or a nitrogen atom).

L₄ is preferably a group consisting of a combination of groups selected from -CONH-, - NHCO-, -COO-, -OCO-, -CO-, -O-, -NH-, and an alkylene group containing 1 to 10 carbon atoms.

L₅ is preferably a triazole group, -S-, -CONH-, -NHCO-, -COO-, -OCO-, -CO-, -O-, -NH-, an alkylene group containing 1 to 10 carbon atoms, or a group consisting of a combination of these groups.

The conjugate of the present invention can be synthesized in accordance with a method comprising allowing Compound 3 described in Examples later (i.e. a biotin-modified dimer having a reactive group at the end thereof) to react with Compound 4 described in Examples later (i.e. a duocarmycin derivative) to synthesize Compound 5.

### (3) Therapeutic kit using conjugate of duocarmycin derivative and biotin-modified dimer

According to the present invention, a therapeutic kit, in which a conjugate of a duocarmycin derivative and a biotin-modified dimer is combined with a mutant streptavidin-molecular probe conjugate, is provided.

As mutant streptavidins used herein, those described in International Publication WO2014/129446 and International Publication WO2015/125820 can be used. Particularly preferably, the mutant streptavidin LISA314-V2122 described in Example 3 of International Publication WO2015/125820 (SEQ ID NO: 4 of International Publication WO2015/125820) (SEQ ID NO: 1 of the description of the present application) can be used.

Examples of the molecular probe used herein may include an antibody, a peptide, a nucleic acid, and an aptamer. Specifically, an antibody, a peptide, a nucleic acid, an aptamer, etc., which target the following antigens specifically expressed in cancer, can be used:
Epiregulin (EREG), ROBO 1, 2, 3 and 4, 1-40-β-amyloid, 4-1BB, 5AC, 5T4, ACVR2B, adenocarcinoma antigen, α-fetoprotein, angiopoetin 2, anthrax toxin, AOC3 (VAP-1), B-lymphoma cells, B7-H3, BAFF, β amyloid, C242 antigen, C5, CA-125, carbonic anhydrase 9 (CA-IX), cardiac myosin, CCL11 (eotaxin-1), CCR4, CCR5, CD11, CD18, CD125, CD140a, CD147 (basigin), CD147 (basigin), CD15, CD152, CD154 (CD40L), CD154, CD19, CD2, CD20, CD200, CD22, CD221, CD23 (IgE receptor), CD25 (IL-2 receptor α chain), CD28, CD3, CD30 (TNFRSF8), CD33, CD37, CD38 (cyclic ADP ribose hydrolase), CD4, CD40, CD41 (integrin α-IIb), CD44 v6, CD5, CD51, CD52, CD56, CD6, CD70, CD74, CD79B, CD80, CEA, CFD, ch4D5, CLDN18.2, *Clostridium difficile,* clumping factor A, CSF2, CTLA-4, cytomegalovirus, cytomegalovirus glycoprotein B, DLL4, DR5, *E*. *coli* Shiga toxin type 1*, E. coli* Shiga toxin type 2, EGFL7, EGFR, endotoxin, EpCAM, episialin, ERBB3, *Escherichia coli,* F protein of respiratory syncytial virus, FAP, fibrin II β chain, fibronectin extra domain-B, folate receptor 1, Frizzled receptor, GD2, GD3 ganglioside, GMCSF receptor α chain, GPNMB, hepatitis B surface antigen, hepatitis B virus, HER1, HER2/neu, HER3, HGF, HIV-1, HLA-DRβ, HNGF, Hsp90, human β amyloid, human scatter factor receptor kinase, human TNF, ICAM-1 (CD54), IFN-α, IFN-γ, IgE, IgE Fc region, IGF-1 receptor, IGF-I, IgG4, IGHE, IL-1β, IL-12, IL-13, IL-17, IL-17A, IL-22, IL-23, IL-4, IL-5, IL-6, IL-6 receptor, IL-9, ILGF2, influenza A hemagglutinin, insulin-like growth factor I receptor, integrin α4, integrin α4β7, integrin α5β1, integrin α7β7, integrin αIIbβ3, integrin αvβ3, integrin γ induced protein, interferon receptor, interferon α/β receptor, ITGA2, ITGB2 (CD18), KIR2D, L-selectin (CD62L), Lewis-Y antigen, LFA-1 (CD11a), lipoteichoic acid, LOXL2, LTA, MCP-1, mesothelin, MS4A1, MUC1, mucin CanAg, myostatin, N-glycolylneuraminic acid, NARP-1, NCA-90 (granulocyte antigen), NGF, NOGO-A, NRP1, *Oryctolagus cuniculus,* OX-40, oxLDL, PCSK9, PD-1, PDCD1, PDGF-R α, phosphatidylserine, prostate cancer cells, *Pseudomonas aeruginosa,* Rabies virus glycoprotein, RANKL, respiratory syncytial virus, RHD, Rh (Rhesus) factor, RON, RTN4, sclerostin, SDC1, selectin P, SLAMF7, SOST, sphingosine-1-phosphate, TAG-72, TEM1, tenascin C, TFPI, TGFβ1, TGFβ2, TGF-β, TNF-α, TRAIL-R1, TRAIL-R2, tumor antigen CTAA16.88, MUC1 tumor-specific glycosylation, TWEAK receptor, TYRP1 (glycoprotein 75), VEGF-A, VEGFR-1, VEGFR2, vimentin, and VWF.

Among the above-described antigens, epiregulin (EREG), CEA, and HER2 are preferable.

A fusion body of a molecular probe such as a cancer antigen-specific antibody molecule and a mutant streptavidin is prepared, and the prepared fusion body is then administered to a patient, so that the mutant streptavidin is allowed to be accumulated specifically in cancer cells. Subsequently, a conjugate of a biotin-modified dimer having an affinity for the above-described mutant streptavidin and a duocarmycin derivative is administered to the patient, so that the phthalocyanine dye can be accumulated exactly in the cancer cells.

Otherwise, in the present invention, a complex is prepared by binding a conjugate of a molecular probe such as a cancer antigen-specific antibody molecule and a mutant streptavidin with a conjugate of a biotin-modified dimer and a duocarmycin derivative, and the thus prepared complex can be administered to a patient.

Various types of molecules can be used as antibodies that are to be bound to the mutant streptavidin. Either a polyclonal antibody or a monoclonal antibody may be used. The subclass of the antibody is not particularly limited. Preferably, IgG, and particularly preferably, IgG₁ is used. Furthermore, the term "antibody" includes all of modified antibodies and antibody fragments. Examples of such an antibody include, but are not limited to: a humanized antibody; a human type antibody; a human antibody; antibodies from various types of animals such as a mouse, a rabbit, a rat, a guinea pig and a monkey; a chimeric antibody between a human antibody and an antibody from a different type of animal; diabody; scFv; Fd; Fab; Fab'; and F(ab)'₂.

The bound body of a mutant streptavidin and an antibody can be obtained by applying a method known to persons skilled in the art. For example, such a bound body can be obtained by a chemical bond method (US5,608,060). Alternatively, DNA encoding the mutant streptavidin is ligated to DNA encoding an antibody, and using an expression vector or the like, the ligated DNA is then expressed in a host cell, so that such a bound body can be obtained in the form of a fusion protein. The DNA encoding the mutant streptavidin may be ligated to the DNA encoding an antibody via DNA encoding a suitable peptide, called a linker. The mutant streptavidin-antibody bound body is desirably produced, while keeping the specific binding force between an antibody and a target molecule.

The present invention will be more specifically described in the following examples. However, these examples are not intended to limit the scope of the present invention.

### Examples

### Example 1: Synthesis of conjugate

### Methyl 6-(2-((tert-butoxycarbonyl)amino)ethoxy)-5,7-dimethoxy-1H-indole-2-carboxylate

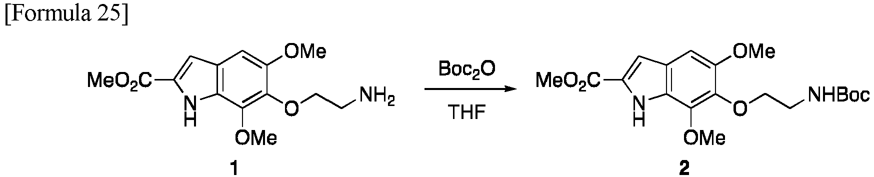

To a solution of compound 1 (Suzawa, T.; Nagamura, S.; Saito, H.; Ohta, S.; Hanai, N.; Yamasaki, M. J. Control. Release 2000, 69, 27) (70.8 mg, 0.241 mmol) in tetrahydrofuran (4.8 mL) was added di*-tert* butyl dicarbonate (106 mg, 0.486 mmol). After stirred under argon atmosphere for 4 hours at room temperature, the solvent was removed under reduced pressure. The crude product was purified by silica gel column chromatography (hexane/ethyl acetate = 5:1 → 1 : 1) to afford compound 2 (92.2 mg, yield: 97%, colorless amorphous).

HRMS (ESI): *m*/*z* 417.1627 [M⁺+Na]

¹H NMR (400 MHz, CDCl₃) δ: 1.38 (s, 9H), 3.31-3.39 (m, 2H), 3.82 (s, 3H), 3.85 (s, 3H), 3.97 (s, 3H), 4.06 (t, 2H, *J* = 5.0 Hz), 5.57 (br s, 1H), 6.76 (s, 1H), 7.02 (d, 1H, *J* = 2.4 Hz), 8.79 (br s, 1H)

### 6-(2-((tert-Butoxycarbonyl)amino)ethoxy)-5,7-dimethoxy-1H-indole-2-carboxylic acid

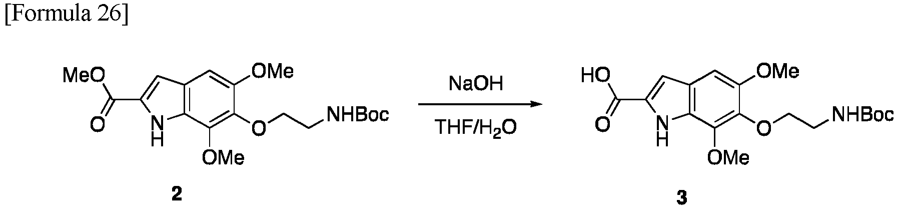

To a solution of compound 2 (233 mg, 0.591 mmol) in tetrahydrofuran (6.0 mL) was added a 1 M aqueous sodium hydroxide (6.0 mL). After stirred under an argon atmosphere for 8.5 h at 50 °C, 1 M hydrochloric acid was added to the reaction mixture at room temperature, and the mixture was extracted with dichloromethane, and the organic layer was then washed with brine. The resultant was dried over sodium sulfate, and the solvent was removed to afford compound 3 (198 mg, yield: 88%, colorless amorphous).

HRMS (ESI): *m*/*z* 381.1642 [M⁺+H]

¹H NMR (400 MHz, CD₃OD) δ: 1.42 (s, 9H), 3.37 (t, 2H, *J=* 5.4 Hz), 3.86 (s, 3H), 4.01 (s, 3H), 4.06 (t, 2H, *J=* 5.4 Hz), 5.57 (br s, 1H), 6.91 (s, 1H), 7.07 (s, 1H)

### tert-Butyl (2-((2-(chlorocarbonyl)-5,7-dimethoxy-1H-indol-6-yl)oxy)ethyl) carbamate

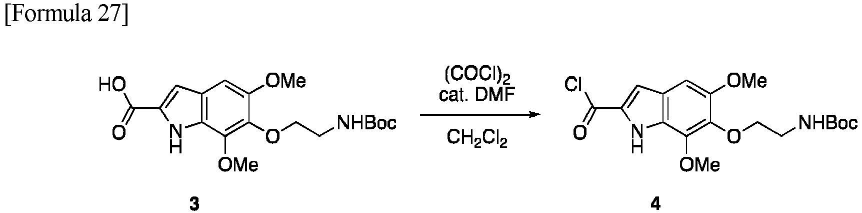

To a solution of compound 3 (120 mg, 0.315 mmol) in dichloromethane (3.2 mL) were added oxalyl chloride (41 µL, 0.47 mmol) and *N,N*-dimethylformamide (1 µL). After stirred under an argon atmosphere at room temperature for 30 minutes, the solvent was removed to provide a crude compound 4.

### 2-(2-(2-Azidoethoxy)ethoxy)ethyl (4-nitrophenyl) carbonate

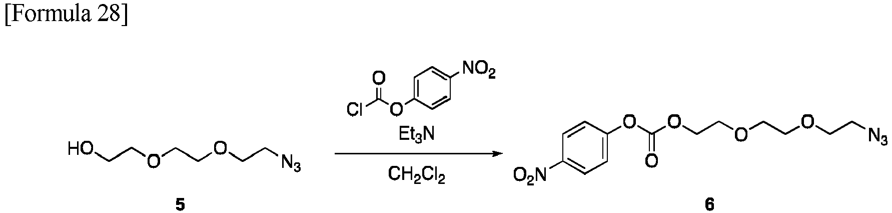

To a solution of alcohol 5 (Dijken, D. J.; Chen, J.; Stuart, M. C. A.; Hou, J.; Feringa, B. L. J. Am. Chem. Soc. 2016, 138, 660) (186 mg, 1.06 mmol) and triethylamine (520 µL, 3.72 mmol) in dichloromethane (5.3 mL) was added 4-nitrophenyl chloroformate (257 mg, 1.28 mmol) at 0°C. After stirred under argon atmosphere at 0 °C for 1.5 hours, dichloromethane was added to the reaction mixture. The organic layer was then washed with saturated aqueous sodium hydrogen carbonate and water, dried over sodium sulfate. The solvent was removed under reduced pressure, and the crude product was purified by silica gel column chromatography (dichloromethane/ethyl acetate = 100 : 0 → 4 : 1) to afford compound 6 (325 mg, yield: 97%, light yellow oil).

HRMS (ESI): *m*/*z* 363.0909 [M⁺+Na]

¹H NMR (400 MHz, CDCl₃) δ: 3.37 (t, 2H, *J=* 5.2 Hz), 3.65-3.73 (m, 6H), 3.80-3.85 (m, 2H), 4.41-4,46 (m, 2H), 7.38 (d, 2H, *J* = 9.2 Hz), 8.26 (d, 2H, *J=* 9.2 Hz)

### Methyl ((2-(2-(2-azidoethoxy)ethoxy)ethoxy)carbonyl)-Z-alaninate

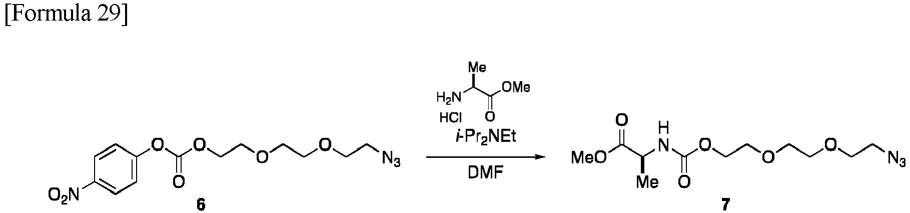

To a solution of carbonate 6 (47.9 mg, 0.151 mmol) and *L*-alanine methyl ester hydrochloride (32.2 mg, 0.231 mmol) in *N,N-*dimethylformamide (760 µL) was added *N,N*-diisopropylethylamine (65 µL, 0.38 mmol) at 0 °C. After stirred under an argon atmosphere at room temperature for 6 hours, the solvent was removed under reduced pressure. The crude product was purified by silica gel column chromatography (hexane/ethyl acetate = 1 : 1 → 1 : 3) to afford compound 7 (39.8 mg, yield: 87%, light yellow oil).

HRMS (ESI): *m*/*z* 327.1283 [M⁺+Na]

¹H NMR (600 MHz, CDCl₃) δ: 1.41 (d, 3H, *J* = 6.6 Hz), 3.40 (t, 2H, *J* = 4.8 Hz), 3.65-3.71 (m, 8H), 3.75 (s, 3H), 4.20-4.27 (m, 2H), 4.36 (quint, 1H, *J* = 7.2 Hz), 5.50 (d, 1H, *J=* 6.0 Hz),

### ((2-(2-(2-Azidoethoxy)ethoxy)ethoxy)carbonyl)-L-alanine

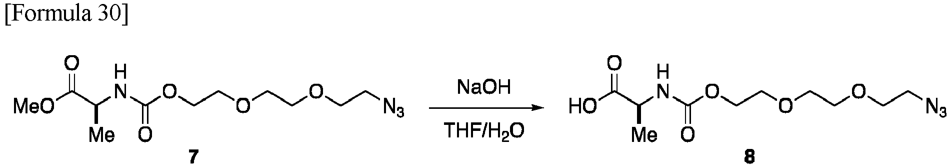

To a solution of compound 7 (12.5 mg, 41.1 µmol) in tetrahydrofuran (1.0 mL) was added 1 M aqueous sodium hydroxide (1.0 mL) at room temperature. After stirred under an argon atmosphere at room temperature for 2.5 hours, 1 M aqueous hydrochloric acid was added to the reaction mixture, and the mixture was extracted with dichloromethane. The organic layer was washed with brine, dried over sodium sulfate. The solvent was removed to provide compound 8 (11.0 mg, yield: 92%, white amorphous).

HRMS (ESI): *m*/*z* 313.1121 [M⁺+Na]

¹H NMR (600 MHz, CD₃OD) δ: 1.38 (d, 3H, *J* = 7.2 Hz), 3.35-3.40 (m, 2H), 3.63-3.71 (m, 8H), 4.13-4.22 (m, 3H, overlapped),

### 2,5-Dioxopyrrolidin-1-yl ((2-(2-(2-azidoethoxy)ethoxy)ethoxy)carbonyl)-L-alaninate

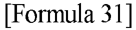

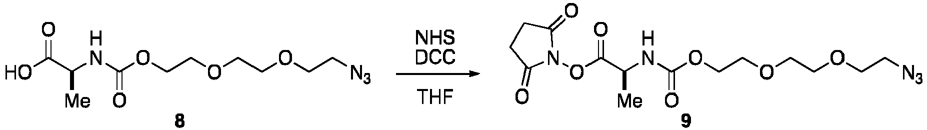

To a solution of carboxylic acid 8 (11.0 mg, 37.9 µmol) in tetrahydrofuran (500 µL) were added *N*-hydroxysuccinimide (4.8 mg, 42 µmol) and *N,N*'-dicyclohexylcarbodiimide (8.6 mg, 42 µmol) at 0 °C. After stirred under argon atmosphere at room temperature for 12 hours, the generated white suspension was filtrated, and the solvent was removed under reduced pressure. The crude product was then purified by silica gel column chromatography (hexane/ethyl acetate = 1 : 1 → 1 : 3) to afford compound 9 (6.6 mg, 42%, colorless oil).

HRMS (ESI): *m*/*z* 410.1276 [M⁺+Na]

¹H NMR (600 MHz, CDCl₃) δ: 1.60 (d, 3H, *J=* 7.8 Hz), 2.85 (s, 4H), 3.39 (t, 2H, *J=* 4.8 Hz), 3.65-3.73 (m, 8H), 4.23-4.32 (m, 2H), 4.70-4.80 (m, 1H), 5.35 (d, 1H, *J=* 6.6 Hz)

### 3-Benzyl-2-methyl-(S)-4-(benzyloxy)-6-(6-(2-((tert-butoxycarbonyl)amino)ethoxy)-5,7-dimethoxy-1H-indole-2-carbonyl)-8-((tert-butyldimethylsilyl)oxy)-6,7,8,9-tetrahydro-3H-pyrrolo[3,2-f]quinoline-2,3-dicarboxylate

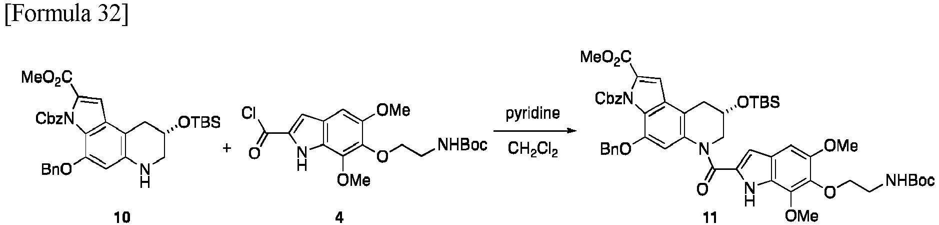

To a solution of pyridine (45 µL, 0.56 mmol) and compound 4 (130 mg, 0.313 mmol) in dichloromethane (4.0 mL) was added compound 10 (Yamada, K.; Kurokawa, T.; Tokuyama, H.; Fukuyama, T. J. Am. Chem. Soc. 2003, 125, 6630; and Okano, K.; Tokuyama, H.; Fukuyama, T. Chem. Asian. J. 2008, 3, 296.) (150 mg, 0.250 mmol) in dichloromethane (2.5 mL) at 0°C. After stirred under argon atmosphere at 0 °C for 5 minutes, 1 M aqueous hydrochloric acid was added to the reaction mixture, and the mixture was extracted with dichloromethane. The organic layer was washed with saturated aqueous sodium hydrogen carbonate, water, and brine, dried over sodium sulfate. The solvent was removed under educed pressure, and the crude product was purified by silica gel column chromatography (hexane/ethyl acetate = 1 : 1) to afford compound 11 (229 mg, yield: 95%, yellow amorphous).

HRMS (ESI): *m*/*z* 985.3979 [M⁺+Na]

¹H NMR (600 MHz, CDCl₃) δ: 0.04 (s, 3H), 0.09 (s, 3H), 0.81 (s, 9H), 1.47 (s, 9H), 2.91 (dd, 1H, *J=* 4.8, 16.2 Hz), 3.24 (dd, 1H, *J=* 6.0, 16.8 Hz), 3.37-3.45 (m, 2H), 3.867 (s, 3H), 3.874 (s, 3H), 4.00 (s, 3H), 4.04-4.15 (m, 3H), 4.33-4.39 (m, 1H), 4.79 (s, 2H), 5.06 (s, 2H), 5.82 (br s, 1H), 6.28 (br s, 1H), 6.71 (s, 1H), 6.76 (br s, 1H), 7.20-7.29 (m, 11H), 9.08 (br s, 1H)

### 3-Benzyl-2-methyl-(S)-4-(benzyloxy)-6-(6-(2-((tert-butoxycarbonyl)amino)ethoxy)-5,7-dimethoxy-1H-indole-2-carbonyl)-8-((methylsulfonyl)oxy)-6,7,8,9-tetrahydro-3H-pyrrolo[3,2-f]quinoline-2,3-dicarboxylate

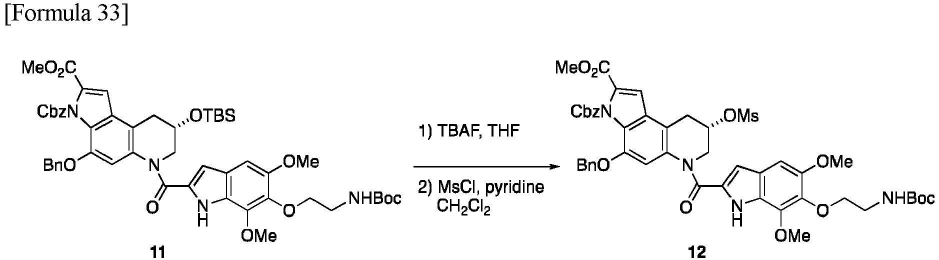

To a solution of compound 11 (88.7 mg, 92.1 µmol) in tetrahydrofuran (1.0 mL) was added tetra-n-butylammonium fluoride (1.0 M tetrahydrofuran solution, 100 µL, 100 µmol) at room temperature. After stirred under argon atmosphere at room temperature for 30 minutes, tetrahydrofuran was removed under reduced pressure. To the crude material in dichloromethane (1.0 mL) were added pyridine (150 µL, 1.86 mmol) and methanesulfonyl chloride (75 µL, 0.97 mmol) at 0 °C. After stirred under argon atmosphere at room temperature for 2.5 hours, the mixture was diluted by ethyl acetate, and the organic layer was washed with 1 M aqueous hydrochloric acid, saturated aqueous sodium hydrogen carbonate, and brine. The organic layer was dried over sodium sulfate, and the solvent was removed under reduced pressure. The crude product was purified by silica gel column chromatography (hexane/ethyl acetate = 1 : 4) to afford compound 12 (52.8 mg, two-step yield: 62%, yellow amorphous).

HRMS (ESI): *m*/*z* 949.2867 [M⁺+Na]

¹H NMR (600 MHz, CDCl₃) δ: 1.39 (s, 9H), 2.86 (s, 3H), 3.25 (dd, 1H, *J=* 3.6, 17.4 Hz), 3.30-3.37 (m, 3H), 3.78 (s, 3H), 3.81 (s, 3H), 3.94 (s, 3H), 4.01-4.08 (m, 2H), 4.53 (br s, 1H), 4.70 (dd, 2H, *J=* 10.8, 19.2 Hz) 5.00 (dd, 2H, *J* = 12.0, 16.2 Hz), 5.32-5.37 (m, 1H), 5.70 (br s, 1H), 6.13 (br s, 1H), 6.61 (s, 1H), 6.64 (s, 1H), 7.10-7.22 (m, 11H), 9.14 (br s, 1H)

### 3-Benzyl-2-methyl-(S)-4-(benzyloxy)-6-(6-(2-((S)-2-(((benzyloxy)carbonyl)amino)-3-methylbutanamido)ethoxy)-5,7-dimethoxy-1H-indole-2-carbonyl)-8-((methylsulfonyl)oxy)-6,7,8,9-tetrahydro-3H-pyrrolo[3,2-f]quinoline-2,3-dicarboxylate

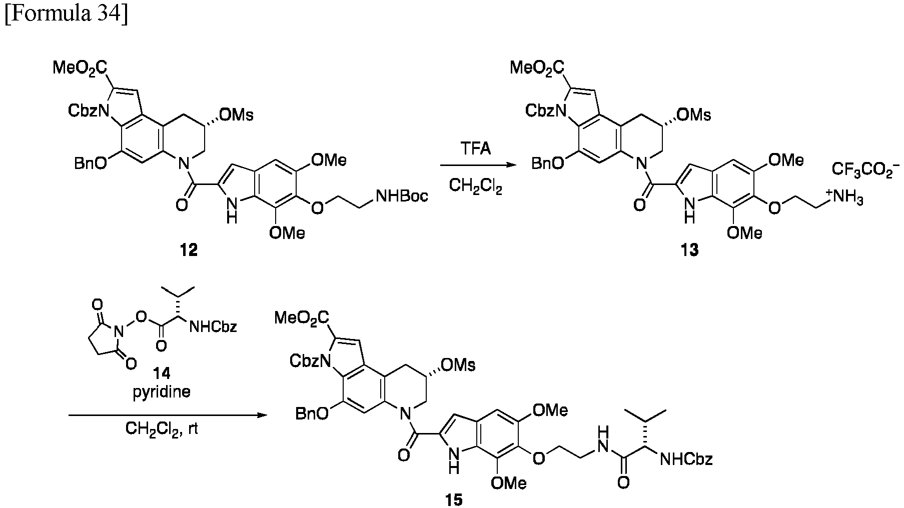

To a solution of carbamate 12 (39.9 mg, 43.0 µmol) in CH₂Cl₂ (2.0 mL) was added trifluoroacetic acid (1.0 mL) at 0 °C. After stirred under argon atmosphere for 30 minutes, the reaction was diluted by dichloromethane and toluene. Then, the solvent was removed under reduced pressure to provide a crude compound **13** (51.0 mg).

An excessive amount of succimide ester 14 in CH₂Cl₂ and pyridine (50 µL) were added to the crude compound **13** (51.0 mg) in CH₂Cl₂ (1.0 mL). After stirred under argon atmosphere at room temperature for 24 hours, the reaction was diluted by ethyl acetate, and the organic layer was washed with 1 M aqueous hydrochloric acid, saturated aqueous sodium hydrogen carbonate, and brine. The mixture was dried over sodium sulfate, and the solvent was removed under reduced pressure to provide a crude material. The crude material was purified by silica gel column chromatography (hexane/ethyl acetate = 1 : 3) to afford compound 15 (41.8 mg, two-step yield: 92%, pink film). HRMS (ESI): *m*/*z* 1060.3533 [M⁺+H]

¹H NMR (600 MHz, CDCl₃) δ: 0.88 (d, 3H, *J=* 6.6 Hz), 0.95 (d, 3H, *J=* 7.2 Hz), 2.08-2.15 (m, 1H), 2.94 (s, 3H), 3.33 (dd, 1H, *J* = 3.6, 17.4 Hz), 3.41 (dd, 1H, *J* = 6.0, 17.4 Hz), 3.51-3.60 (m, 2H), 3.60-3.70 (m, 1H), 3.85 (s, 3H), 3.88 (s, 3H), 3.99 (s, 3H), 4.05 (t, 1H, *J=* 7.2 Hz), 4.10-4.19 (m, 2H), 4.60 (br s, 1H), 4.78 (dd, 2H, *J* = 12.0, 19.2 Hz), 5.07 (d, 2H, *J=* 3.0 Hz), 5.10 (d, 2H, *J=* 3.6 Hz), 5.40-5.45 (m, 1H), 5.47 (d, 1H, *J=* 9.0 Hz), 6.22 (br s, 1H), 6.70 (s, 1H), 6.73 (s, 1H), 7.07 (br s, 1H), 7.20-7.36 (m, 17H), 9.26 (br s, 1H)

### Methyl-(7bR,8aS)-2-(6-(2-((S)-2-amino-3-methylbutanamido)ethoxy)-5,7-dimethoxy-1H-indole-2-carbonyl)-4-oxo-1,2,4,5,8,8a-hexahydrocyclopropa[c]pyrrolo[3,2-e]indole-6-carboxylate

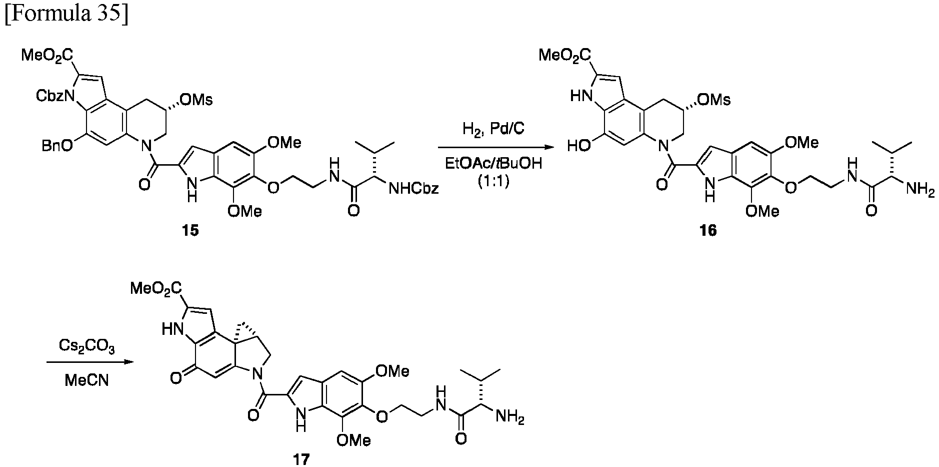

Compound 15 (10.0 mg, 9.43 µmol) and 10% palladium carbon (25.0 mg, 23.5 µmol) in a mixed solution of ethyl acetate (500 µL) and t-butyl alcohol (500 µL) were stirred under hydrogen atmosphere (1 atm) at room temperature for 3.5 hours. The palladium carbon was removed by filtration with Celite, and the residue was then washed with a dichloromethane/methanol (10 : 1) mixed solution. Then, the solvent was removed under reduced pressure to provide a crude compound 16 (5.2 mg, colorless film).

To the crude compound 16 (5.2 mg) and cesium carbonate (30.0 mg, 92.1 µmol) was added toluene, and the solvent was removed under reduced pressure. To the residue was added acetonitrile (1.0 mL), and the mixture was stirred under argon atmosphere at room temperature for 30 minutes. Then, ethyl acetate was added to the generated yellow suspension, and a solid was removed by filtration, followed by washing with dichloromethane. The solvent was removed under reduced pressure to give a crude product. The crude product was purified by preparative thin-layer chromatography (dichloromethane/methanol = 10 : 1) to afford compound 17 (3.1 mg, two-step yield: 54%, yellow film).

HRMS (ESI): *m*/*z* 606.2529 [M⁺+H]

¹H NMR (600 MHz, CDCl₃) δ: 0.84 (d, 3H, *J=* 6.6 Hz), 1.00 (d, 3H, *J=* 6.6 Hz), 1.57 (t, 1H, *J=* 4.8 Hz), 1.76 (dd, 1H, *J* = 4.8, 6.6 Hz), 2.22-2.31 (m, 1H), 2.76-2.82 (m, 1H), 3.26 (d, 1H, *J* = 3.6 Hz), 3.56-3.62 (m, 2H), 3.90 (s, 3H), 3.91 (s, 3H), 4.06 (s, 3H), 4.14-4.22 (m, 2H), 4.39 (d, 1H, *J=* 10.2 Hz), 4.47 (dd, 1H, *J=* 4.8, 9.6 Hz), 6.61 (s, 1H), 6.81 (s, 1H), 6.96 (s, 1H), 7.03 (s, 1H), 7.96 (br s, 1H), 9.36 (br s, 1H)

Methyl-(7b*R*,8a*S*)-2-(6-(((5*S*,8*S*)-19-azido-5-isopropyl-8-methyl-4,7,10-trioxo-11,14,17-trioxa-3,6,9-triazanonadecyl)oxy)-5,7-dimethoxy-1*H*-indole-2-carbonyl)-4-oxo-1,2,4,5,8,8a-hexahydrocyclopropa[*c*]pyrrolo[3,2-*e*]indole-6-carboxylate

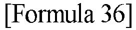

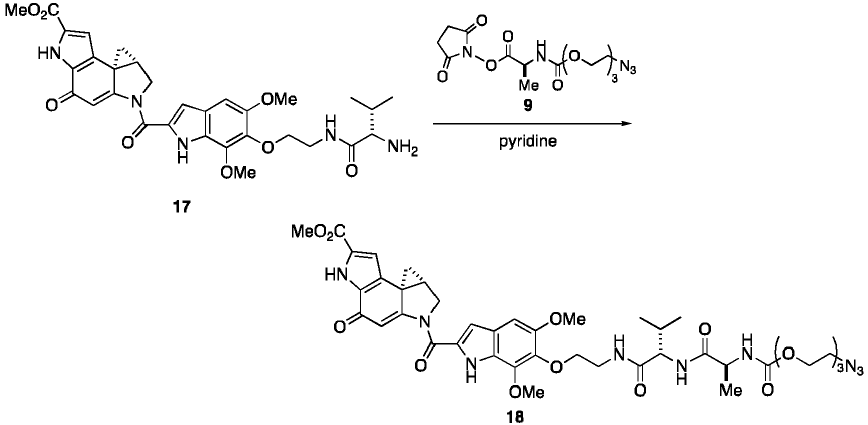

A dichloromethane solution of compound 17 (4.4 mg, 7.3 µmol) and compound 9 (3.4 mg, 8.8 µmol) was distilled away under reduced pressure, and pyridine (0.50 mL) was added to the residue. After stirred under argon atmosphere at 0°C for 1 hour, dichloromethane and toluene were added to the reaction mixture, and the solvent was then removed under reduced pressure. The crude material was purified by preparative thin-layer chromatography (dichloromethane/methanol = 10 : 1) to afford compound 18 (4.6 mg, 72%, yellow film).

HRMS (ESI): *m*/*z* 878.3647 [M⁺+H]

¹H NMR (600 MHz, CDCl₃) δ: 0.88 (d, 3H, *J=* 7.2 Hz), 0.93 (d, 3H, *J=* 6.6 Hz), 1.37 (d, 3H, *J=* 6.6 Hz), 1.57 (t, 1H, *J* = 4.8 Hz), 1.75 (dd, 1H, *J* = 4.8, 7.2 Hz), 2.08-2.18 (m, 1H), 2.76-2.81 (m, 1H), 3.38 (t, 2H, *J=* 5.1 Hz), 3.50-3.62 (m, 2H), 3.64-3.71 (m, 8H), 3.908 (s, 3H), 3.914 (s, 3H), 4.04 (s, 3H), 4.19 (t, 2H, *J=* 4.8 Hz), 4.18-4.31 (m, 3H, overlapped), 4.32-4.37 (m, 1H), 4.38 (d, 1H, *J=* 10.2 Hz), 4.47 (dd, 1H, *J=* 5.4, 10.8 Hz), 5.42 (br s, 1H), 6.60 (s, 1H), 6.71 (d, 1H, *J=* 8.4 Hz), 6.83 (s, 1H), 6.96 (d, 1H, *J* = 1.8 Hz), 7.03 (s, 1H), 7.15 (br s, 1H), 9.61 (br s, 1H), 10.03 (br s, 1H)

### 2-((4-(5-((3aS,4S,6aR)-2-Iminiohexahydro-1H-thieno[3,4-d]imidazol-4-yl)pentanamido)butyl)amino)-N-(2-((4-(5-((3aS,4S,6aR)-2-iminiohexahydro-1H-thieno[3,4-d]imidazol-4-yl)pentanamido)butyl)amino)-2-oxoethyl)-2-oxo-N-(2-(2-(2-(pent-4-ynamido)ethoxy)ethoxy)ethyl)ethan-1-aminium 2,2,2-trifluoroacetate

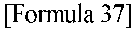

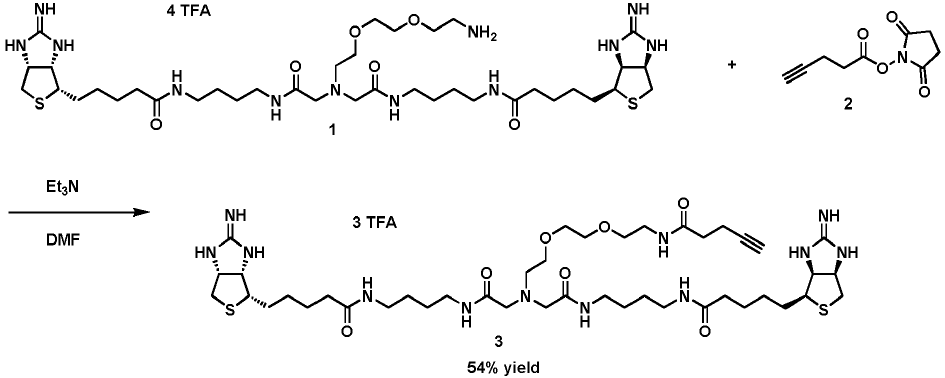

To a solution of bisiminobiotin compound 1 (WO/2020/032165) (5.3 mg, 4.04 µmol) and 2,5-dioxopyrrolidin-1-yl pent-4-ynoate 2 (Funder, E. D.; Jensen, A. B.; Tørring, T.; Kodal, A. L. B.; Azcargorta, A. R.; Gothelf, K. V J. Org. chem. 2012, 77, 3134-3142) (0.95 mg, 4.85 µmol) in DMF (200 µl) was added triethylamine (8.4 µl, 60.6 µmol). After stirred under argon atmosphere at room temperature for 18 hours, the solvent was removed under reduced pressure. The crude product was purified by reverse phase HPLC (gradient: 0% for 5 min; 0-100% for 90 min CH₃CN in 0.1% CF₃COOH aqueous solution, YMC-Triart C18, flow rate = 3.5 ml/min) to afford compound 3 (2.8 mg, yield: 54 %).

LRMS (ESI): *m*/*z* 312.65 [M+3H]³⁺

¹H NMR (500 MHz, CD₃OD) δ:1.40-1.82 (m, 20H), 2.20 (t, *J* = 7.4 Hz, 4H), 2.28 (t, *J* = 2.9 Hz, 1H), 2.38-2.50 (m, 4H), 2.82 (d, *J=* 13.4 Hz, 2H), 3.00 (dd, *J=* 4.6 Hz, 13.2 Hz, 2H), 3.12-3.30 (m, 12H), 3.38 (t, *J=* 5.2 Hz, 2H), 3.55 (t, *J=* 5.7 Hz, 2H), 3,62 (s, 4H), 3.66-3.90 (m, 6H), 4.53 (dd, *J=* 4.6 Hz, 8.0 Hz, 2H), 4.73 (dd, *J=* 4.6 Hz, 8.0 Hz).

### N-(2-(2-(2-(3-(1-((5S,8S)-1-((5,7-Dimethoxy-2-((7bR,8aS)-6-(methoxycarbonyl)-4-oxo-1,2,4,5,8,8a-hexahydrocyclopropa[c]pyrrolo[3,2-e]indole-2-carbonyl)-1H-indol-6-yl)oxy)-5-isopropyl-8-methyl-4,7,10-trioxo-11,14,17-trioxa-3,6,9-triazanonadecan-19-yl)-1H-1,2,3-triazol-4-yl)propanamido)ethoxy)ethoxy)ethyl)-2-((4-(5-((3aS,4S,6aR)-2-iminiohexahydro-1H-thieno[3,4-d]imidazol-4-yl)pentanamido)butyl)amino)-N(2-((4-(5-((3aS,4S,6aR)-2-iminiohexahydro-1H thieno[3,4-d]imidazol-4-yl)pentanamido)butyl)amino)-2-oxoethyl)-2-oxoethan-1-aminium formate

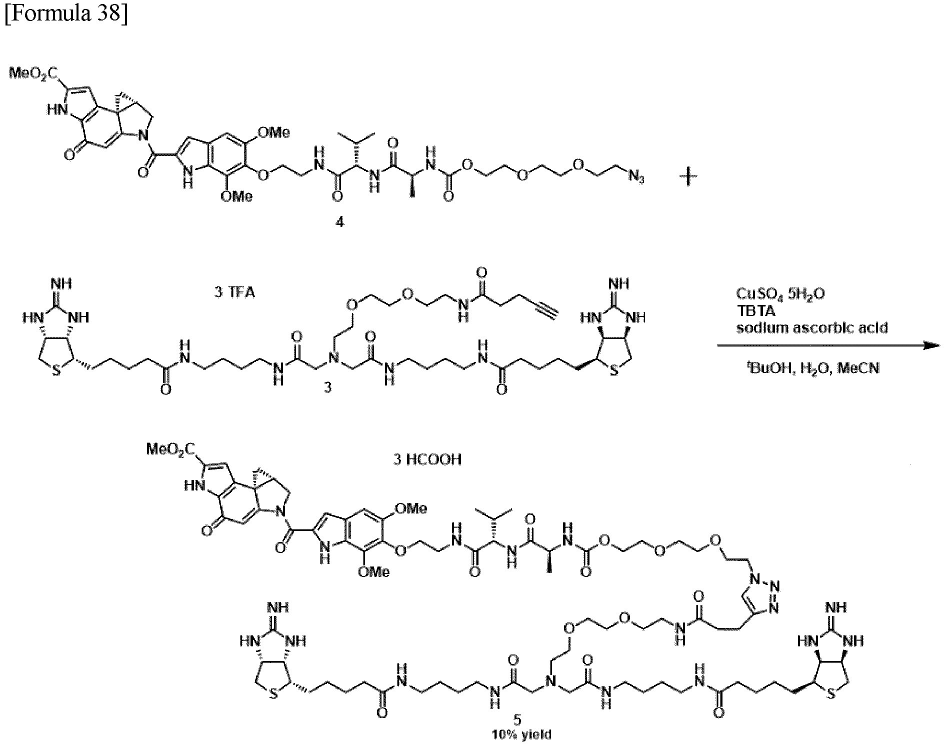

To a solution of compound 3 (2.8 mg, 2.19 µmol) and compound 4 (1.9 mg, 2.19 µmol) in a mixed solution of water (150 µl), acetonitrile (100 µl) and *^{t}*BuOH (150 µl) were added copper(II) sulfate pentahydrate (1.1 mg, 4.38 µmol), tris[(1-benzyl-1*H* 1,2,3-triazol-4-yl)methyl]amine (TBTA, 4.6 mg, 8.76 µmol) and sodium ascorbate (2.6 mg, 13.1 µmol) at room temperature. After stirred under argon atmosphere at room temperature for 3 hours, the reaction mixture was purified by reverse phase HPLC (gradient: 0% for 5 min; 0-100% for 90 min CH₃CN in 0.1% HCOOH aqueous solution, retention time: 40.4 min, YMC-Triart C18, flow rate = 3.5 ml/min) to afford compound 5 (0.4 mg, yield: 10%).

LRMS (ESI): *m*/*z* 605.15 [M+3H]³⁺.

### tert-Butyl (2-((2-(chlorocarbonyl)-1H-indol-5-yl)oxy)ethyl)carbamate

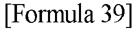

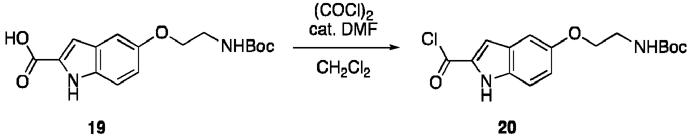

To a solution of compound 19 (20 mg, 0.0624 mmol) in dichloromethane (0.6 mL) were added oxalyl chloride (8.2 µL, 0.0936 mmol) and *N,N-*dimethylformamide (0.2 µL). After stirred under argon atmosphere at room temperature for 30 minutes, the solvent was removed under reduced pressure to afford a crude compound 20.

### 3-Benzyl-2-methyl-(S)-4-(benzyloxy)-6-(5-(2-((,S)-2-(((benzyloxy)carbonyl)amino)-3-methylbutanamido)ethoxy)-1H-indole-2-carbonyl)-8-((methylsulfonyl)oxy)-6,7,8,9-tetrahydro-3H-pyrrolo[3,2-f]quinoline-2,3-dicarboxylate

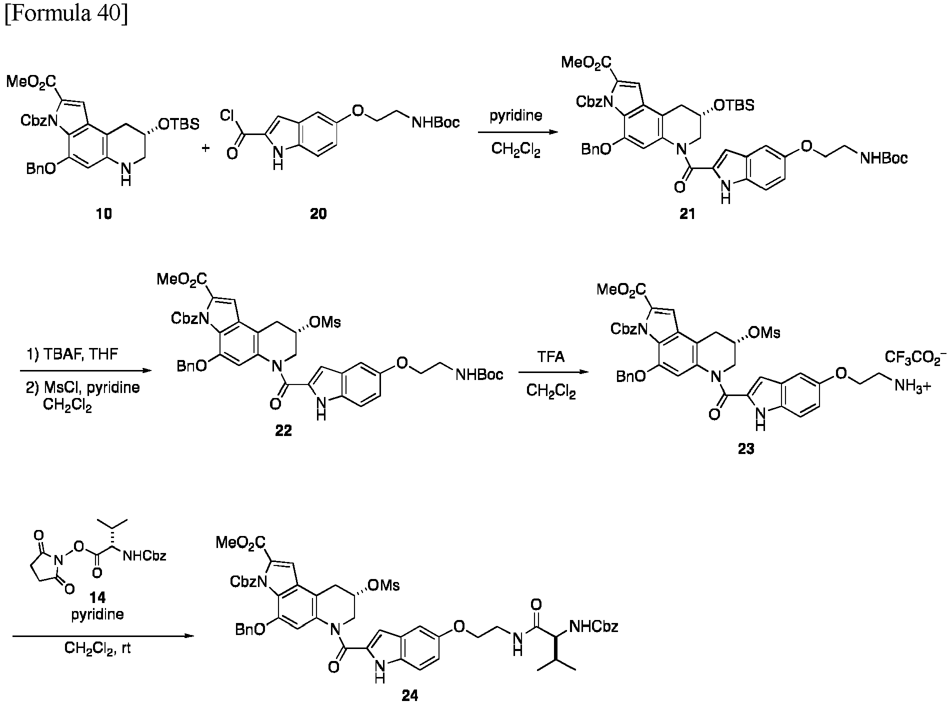

To a solution of compound 10 (31.2 mg, 0.0520 mmol) in dichloromethane (0.6 mL) were added pyridine (10 µL, 0.124 mmol) and the crude compound 20 at 0 °C. After stirred under argon atmosphere at 0 °C for 10 minutes, 1 M aqueous hydrochloric acid was added to the reaction mixture, and the mixture was extracted with dichloromethane. The organic layer was then washed with saturated aqueous sodium hydrogen carbonate, water, and brine, dried over sodium sulfate, and the solvent was removed under reduced pressure. The crude product was purified by silica gel column chromatography (hexane/ethyl acetate = 3 : 1) to afford compound 21 (36.2 mg) with unidentified impurities.

To a solution of compound 21 (36.2 mg) in tetrahydrofuran (1.0 mL) was added tetra-n-butylammonium fluoride (1.0 M tetrahydrofuran solution, 44 µL, 44 µmol) at room temperature. After stirred under argon atmosphere at room temperature for 30 minutes, tetrahydrofuran was removed under reduced pressure to give a crude material.

To a solution of the crude material in a dichloromethane (0.6 mL) were added pyridine (75 µL, 0.93mmol) and methanesulfonyl chloride (40 µL, 0.517 mmol) at 0 °C. After stirred under argon atmosphere at room temperature for 2.5 hours, ethyl acetate was added to the reaction mixture, and the organic layer was washed with 1 M aqueous hydrochloric acid, saturated aqueous sodium hydrogen carbonate, and brine. The resultant was dried over sodium sulfate, and the solvent was removed under reduced pressure. The crude product was purified by silica gel column chromatography (dichloromethane/methanol = 9 : 1) to afford compound 22 (59.6 mg) with unidentified impurities.

To a solution of compound 22 (59.6 mg) in dichloromethane (2.0 mL) was added trifluoroacetic acid (1.0 mL) at 0 °C. After stirred under argon atmosphere for 30 minutes at room temperature, dichloromethane and toluene were added to the reaction mixture, and the solvent was removed under reduced pressure to afford a crude compound 23.

To a solution of the crude compound 23 in dichloromethane (1.5 mL) were added an excessive amount of succimide ester 14 in dichloromethane and pyridine at 0 °C. After stirred for argon atmosphere at room temperature for 3 hours, dichloromethane was added to the reaction mixture, and the organic layer was washed with 1 M aqueous hydrochloric acid, saturated aqueous sodium hydrogen carbonate, and brine. The resultant was dried over sodium sulfate, and the solvent was removed under reduced pressure to give a crude material. The crude material was purified by silica gel column chromatography (hexane/ethyl acetate = 1 : 1) to afford compound 24 (29.2.8 mg, two-step yield: 73%, colorless powder).

### Methyl (7bR,8aS)-2-(5-(2-((S)-2-amino-3-methylbutanatnido)ethoxy)-1H-indole-2-carbonyl)-4-oxo-1,2,4,5,8,8a-hexahydrocyclopropa[c]pyrrolo[3,2-e]indole-6-carboxylate

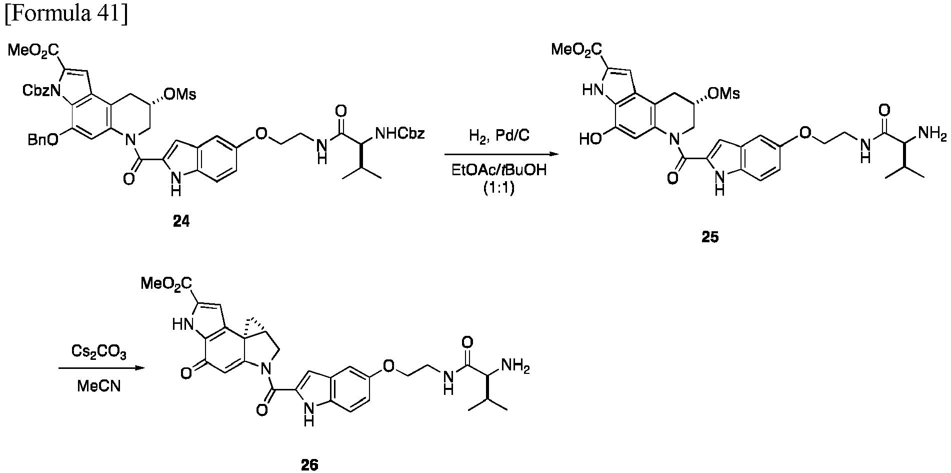

Compound 24 (20.0 mg, 19.9 µmol) and 10% palladium carbon (50.0 mg, 47.0 µmol) in a mixed solution of ethyl acetate (1.5 mL) and *t*-butyl alcohol (1.5 mL) were stirred under hydrogen atmosphere (1 atm) at room temperature for 5 hours. Palladium carbon was removed by filtration with Celite, and the residue was washed with a mixed solution of dichloromethane and methanol (10 : 1). Then, the solvent was removed under reduced pressure to afford a crude compound 25.

To the crude compound 25 and cesium carbonate (60.0 mg, 184 µmol) was added toluene, and the solvent was removed under reduced pressure. To the residue was added acetonitrile (2.0 mL) and the mixture was stirred under argon atmosphere at room temperature for 30 minutes. Then, ethyl acetate was added to the generated yellow suspension, and the solid was removed by filtration, followed by washing with dichloromethane. The solvent was removed under reduced pressure to give a crude product. The crude product was purified by preparative thin-layer chromatography (dichloromethane/methanol = 9 : 1) to afford compound 26 (3.5 mg, two-step yield: 32%, colorless powder).

HRMS (ESI): *m*/*z* 546.2320 [M⁺+H]

¹H NMR (400 MHz, CD₃OD) δ: 0.94 (d, 3H, *J=* 6.8 Hz), 0.97 (d, 3H, *J=* 6.8 Hz), 1.60 (t, 1H, *J=* 4.4 Hz), 1.85 (dd, 1H, *J* = 4.4, 7.6 Hz), 1.96-2.00 (m, 1H), 2.99-3.03 (m, 1H), 3.22 (d, 1H, *J* = 6.0 Hz), 3.60 (ddd, 1H, *J=* 5.2, 5.2, 14.0 Hz), 3.69 (ddd, 1H, *J=* 5.2, 5.2, 14.0 Hz), 3.88 (s, 3H), 4.08 (m, 2H), 4.48 (d, 1H, *J=* 10.8 Hz), 4.53 (dd, 1H, *J=* 52, 10.8 Hz), 6.73 (s, 1H), 6.81 (s, 1H), 6.97 (dd, 1H, *J=* 2.0, 8.8 Hz),7.07 (s, 1H), 7.12 (d, 1H, *J=* 2.0 Hz), 7.35 (d, 1H, *J=* 8.8 Hz)

### Methyl (7bR,8aS)-2-(5-(((6S,9S)-1-azido-9-isopropyl-6-methyl-4,7,10-trioxo-3-oxa-5,8,11-triazatridecan-13-yl)oxy)-1H-indole-2-carbonyl)-4-oxo-1,2,4,5,8,8a-hexahydrocyclopropa[c]pyrrolo[3,2-e]indole-6-carboxylate

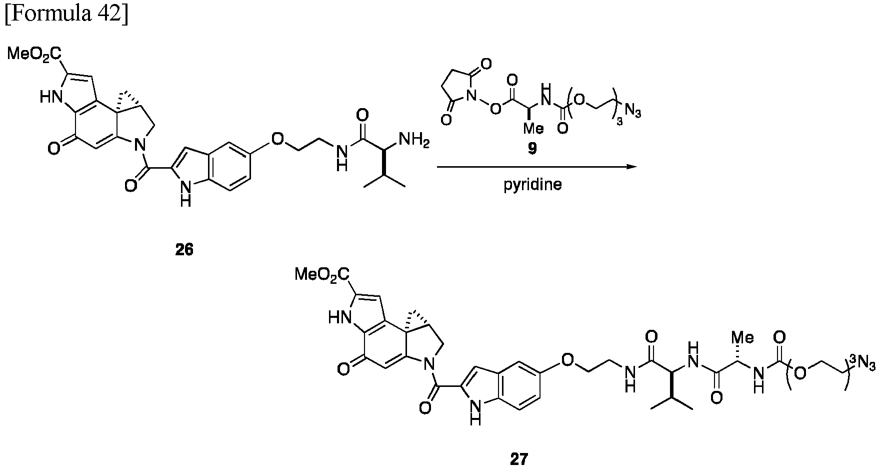

A dichloromethane solution of compound 26 (3.0 mg, 5.5 µmol) and compound 9 (2.6 mg, 6.72 µmol) was distilled away under reduced pressure, and pyridine (0.50 mL) was added to the residue. After stirred under argon atmosphere at 0 °C for 30 minutes, dichloromethane and toluene were added to the reaction mixture, and the solvent was t removed under reduced pressure. The crude product was purified by preparative thin-layer chromatography (dichloromethane/methanol = 9 : 1) to afford compound 27 (4.0 mg, 89%, colorless powder).

HRMS (ESI): *m*/*z* 818.3444 [M⁺+H]

¹H NMR (400 MHz, CD₃Cl₃) δ: 0.92 (d, 3H, *J* = 6.8 Hz), 0.94 (d, 3H, *J* = 6.8 Hz),

1.33 (d, 3H, *J* = 7.2 Hz), 1.54 (t, 1H, *J* = 4.4 Hz), 1.72-1.75 (m, 1H), 2.16-2.21 (m, 1H), 2.77-2.81 (m, 1H), 3.38 (t, 2H, *J=* 5.8 Hz), 3.65-3.68 (m, 10H), 3.91 (s, 3H), 4.03-4.05 (m, 2H), 4.19-4.26 (m, 3H), 4.3 (dd, 1H, *J* = 6.4, 8.8 Hz), 4.39-4.49 (m, 2H), 5.41 (d, 1H, *J* = 6.8 Hz), 6.59 (s, 1H), 6.68 (br, 1H), 6.76 (dd, 1H, *J* = 8.4 Hz), 6.93-6.99 (m, 3H), 7.08 (s, 1H), 7.32 (d, 1H, *J=* 9.2 Hz), 79.57 (s, 1H), 10.0 (br s, 1H).

### Example 2: Expression and purification of CEA-V2122 protein

V2122 is the mutant streptavidin described in Example 3 of International Publication WO2015/125820 (SEQ ID NO: 4 shown in International Publication WO2015/125820). The amino acid sequence of V2122 (a sequence having a 6xHis tag at the C-terminus) is as set forth in SEQ ID NO: 1 in the sequence listing.

scFv-V2122 is prepared by binding a single-chain antibody (scFv) against CEACAM5 with the above-described V2122. This scFv-type anti-CEACAM5 antibody is an scFv sequence described in a patent document US7626011B2. The amino acid sequence of the scFv-type anti-CEACAM5 antibody is as set forth in SEQ ID NO: 2 in the sequence listing. In addition, the amino acid sequence of CEA-V2122 prepared by binding the scFv-type anti-CEACAM5 antibody with V2122 via an amino acid linker (GGGGSGGGG) (SEQ ID NO: 7) is as set forth in SEQ ID NO: 3 in the sequence listing.

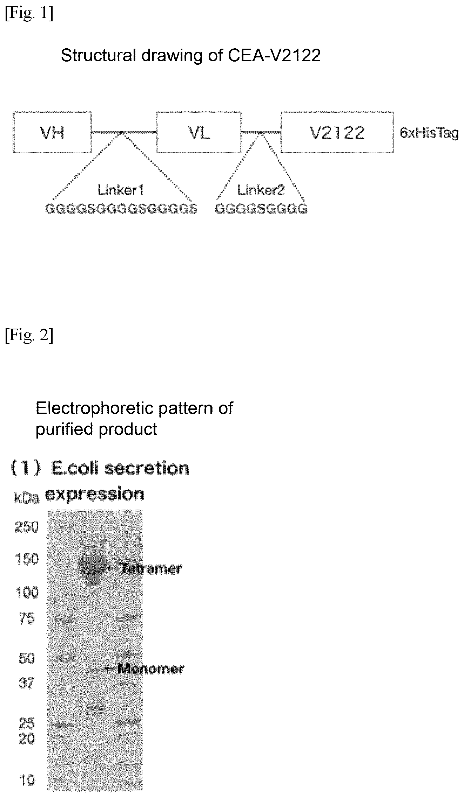

For the expression of a CEA-V2122 fusion protein, the DNA codon of a CEA-V2122 gene sequence, in which a pelB signal for secretion and expression in *Escherichia coli* had been incorporated into the N-terminus, and a 6xHis-Tag sequence had been incorporated into the C-terminus, was optimized for *Escherichia coli,* thereby synthesizing an artificial gene. This amino acid sequence is as set forth in SEQ ID NO: 4 in the sequence listing and the DNA sequence is as set forth in SEQ ID NO: 5 in the sequence listing. Moreover, an outline of a domain structure is shown in Fig. 1.

A vector prepared by incorporating a chaperone skp gene into MCS2 of a pETDuet1 vector was used as a specific protein expression vector. Regarding the skp gene, the DNA codon was optimized for *Escherichia coli* based on the amino acid sequence set forth in SEQ ID NO: 6 in the sequence listing, thereby synthesizing an artificial gene. The synthesized skp gene was amplified by PCR, using the primers (AAGGAGATATACATATGGATAAAATTGCCATTGTTAATAT (SEQ ID NO: 8), and TTGAGATCTGCCATATGTTATTTCACTTGTTTCAGAACG (SEQ ID NO: 9)), and the amplified gene was then cloned into MCS2 of the pETDue1 vector linearized with the restriction enzyme NdeI, using In-Fusion HD Cloning Kit, so as to obtain pETDuet_skp. Subsequently, the CEA-V2122 gene was incorporated into MCS1 of pETDuet_skp. Specifically, the artificially synthesized CEA-V2122 gene was amplified by PCR, using the primers (AGAAGGAGATATACCATGAAATATCTGCTGCCGAC (SEQ ID NO: 10), and CGCCGAGCTCGAATTTTAATGATGGTGATGATGATG (SEQ ID NO: 11)). Moreover, pETDuet_skp was linearized by PCR, using the primers (GGTATATCTCCTTCTTAAAGTTAAAC (SEQ ID NO: 12), and AATTCGAGCTCGGCGCGCCTGCAG (SEQ ID NO: 13)). The CEA-V2122 amplified by PCR and the linearized pETDuet_skp were subjected to cloning using In-Fusion HD Cloning Kit. The cloned vector was confirmed by sequencing in terms of the gene sequence incorporated therein, and thereafter, it was referred to as pETDuet_CEA-V2122_skp.

For the expression of the protein, pETDuet_CEA-V2122_skp was transformed into BL21(DE3) (Nippon Gene Co., Ltd.), which was then pre-cultured in 2xYT medium (SIGMA-ADLRICH) at 37°C overnight. The medium used in the pre-culture was added to a new medium for 100-fold dilution, and culture was then carried out at 37°C until OD (600 nm) became 0.5 to 2.0. Subsequently, IPTG was added to the culture to a final concentration of 0.5 mM, and the obtained mixture was then cultured at 37°C for 4 hours. Thereafter, the culture supernatant was recovered and was then preserved at 4°C.

The CEA-V2122 protein was roughly purified according to a batch method utilizing 6xHis-Tag added to the C-terminus. Specifically, cOmplete His-Tag Purification Resin equilibrated with buffer A (50 mM Tris-HCl, 0.2 M NaCl, 1 mM EDTA, and 5 mM Imidazole; pH 8.0) was added to the culture supernatant preserved at 4°C. The obtained mixture was stirred for 2 hours to overnight at 4°C, so that the protein was allowed to bind to the resin. Subsequently, the resin was recovered into a column, and a 20 column volume of washing operation was performed with buffer A. Thereafter, a roughly purified product of CEA-V2122 was recovered by elution with buffer B (50 mM Tris-HCl, 0.2 M NaCl, 1 mM EDTA, and 400 mM Imidazole; pH 8.0).

Subsequently, the roughly purified product was purified using a Protein L column. Specifically, 1 mL of Capto L (GE Healthcare Life Sciences) was filled into a PD-10 column, and was then equilibrated with 10 column volumes of PBS. The aforementioned roughly purified product was then applied to it. Thereafter, the resultant was washed with 10 column volumes of PBS, was then eluted with 10 mM glycine hydrochloride (pH 2.0), and was then subjected to centrifugal concentration using Vivaspin Turbo 15 (MWCO 100,000). Moreover, using PD-10 (GE Healthcare Life Science), the buffer was replaced with PBS, and centrifugal concentration was further carried out using Vivaspin Turbo 4 (MWCO 100,000) to obtain a finally purified product. After completion of SDS-PAGE electrophoresis, the purity of tetramer CEA-V2122 was assayed by CBB staining. The results are shown in Fig. 2. As an SDS-PAGE gel, Mini-PROTEAN TGX 4-15% (Bio-Rad) was used, and as a CBB staining solution, Bullet CBB Stain One (Ready To Use) (Nacalai Tesque, Inc.) was used.

From Fig. 2, it was confirmed that the purified CEA-V2122 comprises an approximately 150 kDa tetramer as the main component.

### Example 3: Evaluation of the performance of CEA-V2122 by SPR

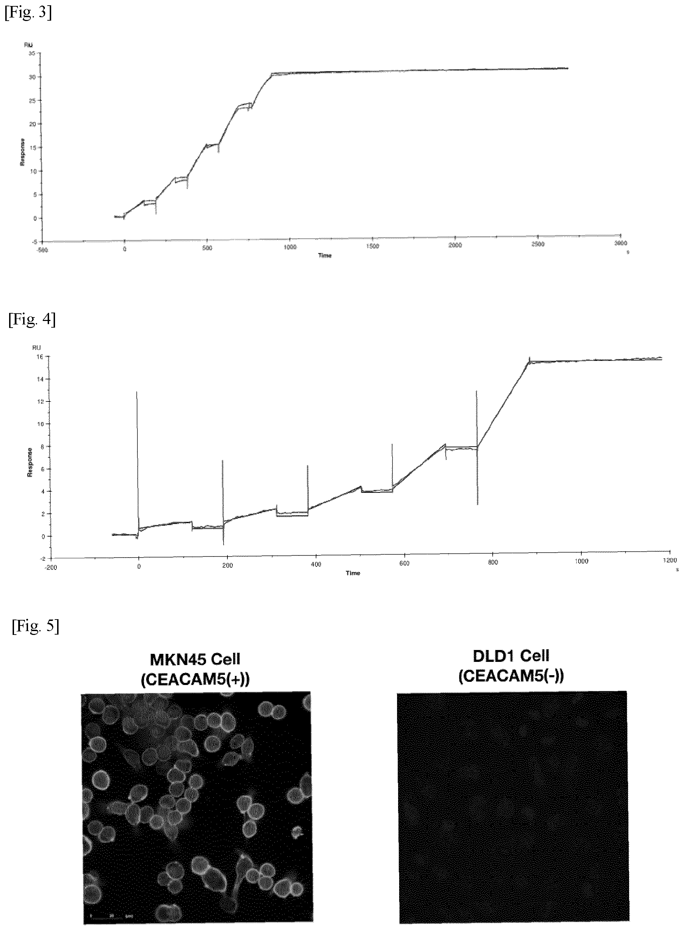

The affinity of CEA-V2122 for the antigen CEACAM5 was evaluated using a surface plasmon resonance (SPR) measuring device, Biacore T200 (GE Healthcare Life Sciences). Specifically, Recombinant Human CEACAM-5/CD66e Protein, CF (R & D SYSTEMS) was immobilized on Sensor Chip CM5 (GE Healthcare Life Sciences) using an amine-coupling kit (GE Healthcare Life Sciences). The final amount of the ligand immobilized was 279 RU. Moreover, about the purified CEA-V2122, two-fold serial dilutions from 1E-08 M to 6.25E-10 M were prepared as analytes. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.208E + 5, and kd1 = 3.461E - 7. Moreover, since evaluation can be carried out at K_{D} = kd1/ka1 in the bivalent analysis, the evaluation value K_{D} = kd1/ka1 = 3.461E - 7 / 3.208E + 5 = 1.078E - 12 was obtained. These results are shown in Fig. 3.

The KD value in the sensorgram shown in Fig. 3 confirmed that CEA-V2122 strongly binds to CEACAM5.

Furthermore, the interaction between CEA-V2122 and a modified biotin was also analyzed using Biacore T200. The modified biotin was specifically the title compound 14 described in Example 1 of International Publication WO2018/07239. In addition, the analysis method was specifically as follows. An amine-coupling kit was used, a target value was set to be 5000 RU, and the purified CEA-V2122 was immobilized on Sensor Chip CM5. With regard to the concentrations of the analytes, 5 types of two-fold serial dilutions from 1E-08 M to 6.25E-10 M were used. Regarding interaction analysis, data were obtained by single-cycle kinetics analysis. Using Biacore T200 Evaluation Software, version 2.0, the obtained data were subjected to curve fitting in a bivalent analysis mode, and the following values were obtained: ka1 = 3.792E + 4, and kd1 = 4.424E - 6. Moreover, since evaluation can be carried out at K_{D} = kd1/ka1 in the bivalent analysis, the evaluation value K_{D} = kd1/ka1 = 3.792E + 4/4.424E - 6 = 1.167E - 10 was obtained. These results are shown in Fig. 4.

The KD value in the sensorgram shown in Fig. 4 confirmed that CEA-V2122 strongly binds to modified biotin.

### Example 4: Cell staining of CEACAMS-expressing cell line using FITC-labeled CEA-V2122

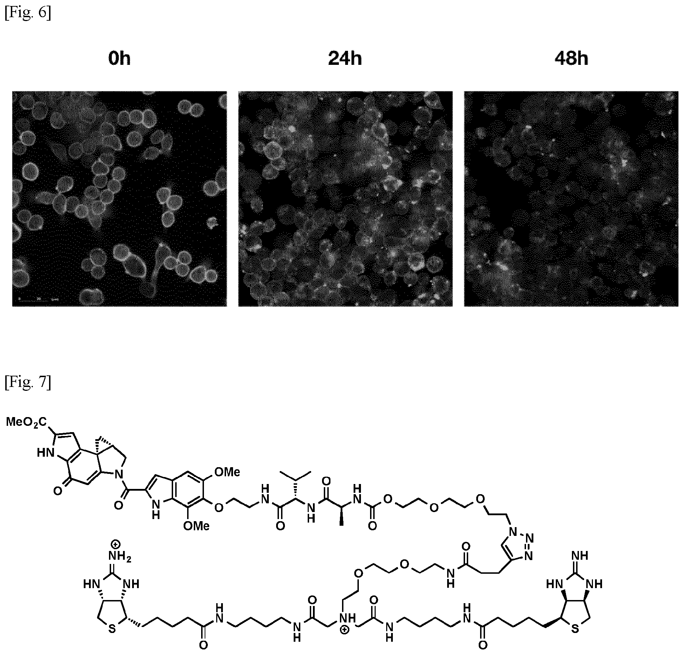

In order to stain a CEACAM5 expression-positive cancer cell line, FITC labeling was carried out on the cell line, using 100 µg of a purified CEA-V2122 protein. Specifically, labeling was carried out using Fluorescein Labeling Kit - NH₂ (DOJINDO LABORATORIES) in accordance with the dosage and administration described in the operating manual included with the kit, and the obtained product was defined to be CEA-V2122-FITC. Specific staining of the CEACAM5 expression-positive cancer cell line is as follows. That is, CEACAM5-positive human stomach cancer-derived MKN-45 cells and CEACAM5-negative human colon cancer-derived DLD1 cells were each seeded on a CELLSTAR, µClear, 96-well plate (Greiner) to a cell density of 2.0 × 10⁴ cells/well, and thereafter, the cells were cultured overnight. Subsequently, a culture solution containing 20 nM CEA-V2122-FITC and 1 µM Hoechist was added to the 96-well plate to a concentration of 100 µL/well, and the obtained mixture was then reacted at 4°C for 30 minutes. Thereafter, each image was taken using In Cell Analyzer 6000 (GE Healthcare Life Sciences). The results are shown in Fig. 5 and Fig. 6.

From the results shown in Fig. 5, it was confirmed that CEA-V2122-FITC specifically recognizes CEACAM5 on the surface of the cell membrane. In addition, from the results shown in Fig. 6, it was confirmed that after CEA-V2122-FITC has bound to CEACAM5, the CEACAM5 stays on the surface of the cell membrane.

### Example 5: In vitro cytotoxicity test using CEA-V2122 and anticancer agent-labeled modified biotin

A cytotoxicity test was carried out using an anticancer agent-labeled modified biotin. The anticancer agent-labeled modified biotin is a compound described as Compound 5. Compound 5 is shown in Fig. 7. For the assay, first, CEACAM5-positive MKN45 cells and CEACAM5-negative DLD1 cells were seeded on a 96-well plate for cell culture, so that the cell count became 5 × 10³ cells/well and the amount of the culture solution became 50 µL/well, and thereafter, the cells were cultured overnight. A solution containing a complex of CEA-V2122 and a photoactivatable compound-labeled modified biotin was prepared by mixing CEA-V2122 and Compound 5 to a molar ratio of 1 : 2, and then incubating the mixture at room temperature for 10 minutes. Thereafter, the concentration of the reaction solution was adjusted with a culture solution, so that the final concentration of Compound 5 became 1200 nM. Regarding serial dilutions, 3-fold serial dilutions were prepared from a mixed solution containing CEA-V2122 (concentration: 600 nM) and Compound 5 (concentration: 1200 nM) for 11 series (i.e. 2.0E-11, 6.1E-11, 1.8E-10, 5.5E-10, 1.6E-09, 4.9E-09, 1.5E-08, 4.4E-07, 1.3E-07, 4.0E-07, and 1.2E-06), and these series were used as complex serial dilution solutions. In addition, a medium alone that did not contain complexes was used as a zero control.

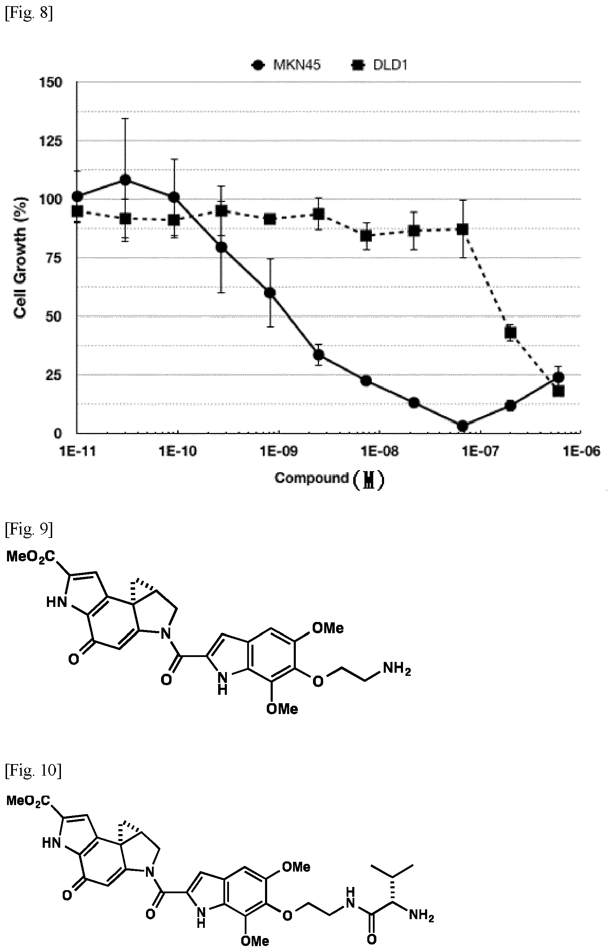

To the cells that had been cultured overnight, the complex serial dilution solutions were added in an amount of 50 µL/well to each well of the plate to the final molar concentration (1.0E-11, 3.0E-11, 9.1E-11, 2.7E-10, 8.2E-10, 2.5E-09, 7.4E-09, 2.2E-08, 6.7E-08, 2.0E-07, and 6.0E-07). After completion of the addition of the complex serial dilution solutions, the cells were cultured for 48 hours. Thereafter, a comparison was made in terms of the number of surviving cells, using Cell Counting Kit-8 (DOJINDO LABORATORIES). Each condition was set to be n = 5. The dosage and administration were determined by the instruction manuals included with the kit, and after the addition of the reagent, the mixture was incubated for 1.5 hours at 37°C in a COz incubator. Thereafter, the absorbance at 450 nm was measured, and a mean value was then calculated, followed by background collection. The control was set to 100%, and the cell growth ratio under each condition to the control was calculated. The results obtained by repeating this experiment three times and then graphically showing the mean value and standard deviation are shown in Fig. 8.

As shown in Fig. 8, suppression of the cell growth of the CEACAM5-positive MKN45 cells was confirmed in a complex concentration-dependent manner. On the other hand, such a concentration-dependent effect of suppressing the cell growth of the CEACAM5-negative DLD1 cells was not confirmed.

### Example 6: Cytotoxicity of Duocarmycin SA and Duocarmycin SA-Valine

In order to confirm the activities of Compound X (Duocarmycin SA) and Compound Y (Duocarmycin SA-Valin) shown in Fig. 9 and Fig. 10, cytotoxicity was confirmed using HeLa S3 cells.

The cells were seeded on a 96-well plate for cell culture, so that the cell count became 5 × 10³ cells/well and the amount of the culture solution became 50 µL/well, and thereafter, the cells were cultured overnight. The concentration of Compound X or Compound Y was adjusted with a culture solution, so that the final concentration thereof became 600 nM. Regarding serial dilutions, 3-fold serial dilutions were prepared from a solution containing Compound X or Compound Y (concentration: 600 nM) for 11 series (i.e. 1.0E-11, 3.0E-11, 9.1E-11, 2.7E-10, 8.2E-10, 2.5E-09, 7.4E-09, 2.2E-08, 6.7E-08, 2.0E-07, 6.0E-07), and these series were used as agent-diluted solutions. In addition, a medium alone that did not contain compounds was used as a zero control.

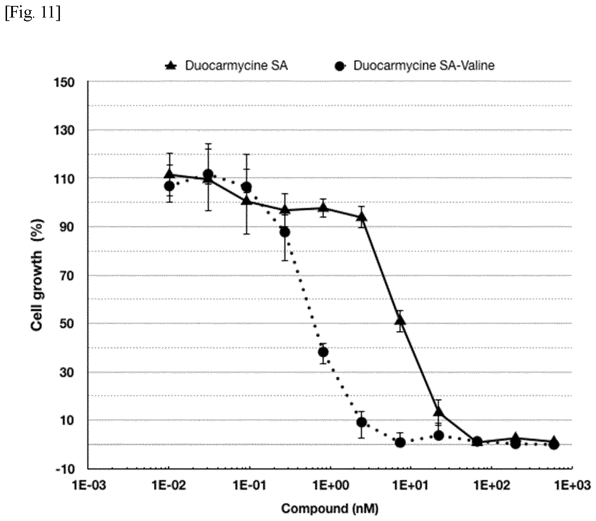

The medium used in the overnight culture of the cells was exchanged with the prepared agent-diluted solution, and the cells were then cultured for 48 hours. Thereafter, a comparison was made in terms of the number of surviving cells, using Cell Counting Kit-8 (DOJINDO LABORATORIES). Each condition was set to be n = 3. The dosage and administration applied in the assay were determined in accordance with the instruction manuals included with the kit, and after addition of the reagent, the mixture was incubated for 1.5 hours at 37°C, in a CO₂ incubator. Thereafter, the absorbance at 450 nm was measured, and a mean value was then calculated, followed by background collection. The control was set to be 100%, and the ratio of the cell growth under each condition to the control was calculated. The results obtained by repeating this experiment three times and then graphically showing the mean value and standard deviation are shown in Fig. 11.

From Fig. 11, it was confirmed that Duocarmycin SA-Valine has a higher effect of suppressing cell growth than Duocarmycin SA.
SEQ ID NO:1 
AEAGITGTWSDQLGDTFIVTAGADGALTGTYENAVGGAESRYVLTGRYDSAPATDGSGTA
LGWTVAWKNNSKNAHSATTWSGQYVGGADAKINTQWLLTSGTTNANAWKSTLVGHDT
FTKVKPSAASHHHHHH
SEQ ID NO:2(sm3E-scFv sequence) 
QVKLEQSGAEVVKPGASVKLSCKASGFNIKDSYMHWLRQGPGQRLEWIGWIDPENGDT
EYAPKFQGKATFTTDTSANTAYLGLSSLRPEDTAVYYCNEGTPTGPYYFDYWGQGTLVTV
SSGGGGSGGGGSGGGGSENVLTQSPSSMSVSVGDRVNIACSASSSVPYMHWLQQKPGKS
PKLLIYLTSNLASGVPSRFSGSGSGTDYSLTISSVQPEDAATYYCQQRSSYPLTFGGGTKLEI
K
SEQ ID NO:3 
QVKLEQSGAEVVKPGASVKLSCKASGFNIKDSYMHWLRQGPGQRLEWIGWIDPENGDT
EYAPKFQGKATFTTDTSANTAYLGLSSLRPEDTAVYYCNEGTPTGPYYFDYWGQGTLVT
VSSGGGGSGGGGSGGGGSENVLTQSPSSMSVSVGDRVNIACSASSSVPYMHWLQQKPGK
SPKLLIYLTSNLASGVPSRFSGSGSGTDYSLTISSVQPEDAATYYCQQRSSYPLTFGGGTKL
EIKGGGGSGGGGAEAGITGTWSDQLGDTFIVTAGADGALTGTYENAVGGAESRYVLTGR
YDSAPATDGSGTALGWTVAWKNNSKNAHSATTWSGQYVGGADAKINTQWLLTSGTTN
ANAWKSTLVGHDTFTKVKPSAASHHHHHH
SEQ ID NO:4 
MKYLLPTAAAGLLLLAAQPAMAQVKLEQSGAEVVKPGASVKLSCKASGFNIKDSYMH
WLRQGPGQRLEWIGWIDPENGDTEYAPKFQGKATFTTDTSANTAYLGLSSLRPEDTAVY
YCNEGTPTGPYYFDYWGQGTLVTVSSGGGGSGGGGSGGGGSENVLTQSPSSMSVSVGD
RVNIACSASSSVPYMHWLQQKPGKSPKLLIYLTSNLASGVPSRFSGSGSGTDYSLTISSVQ
PEDAATYYCQQRSSYPLTFGGGTKLEIKGGGGSGGGGAEAGITGTWSDQLGDTFIVTAG
ADGALTGTYENAVGGAESRYVLTGRYDSAPATDGSGTALGWTVAWKNNSKNAHSATT
WSGQYVGGADAKINTQWLLTSGTTNANAWKSTLVGHDTFTKVKPSAASHHHHHH
SEQ ID NO:5 
ATGAAATATCTGCTGCCGACCGCAGCAGCGGGTCTGCTGCTGCTGGCAGCACAGCCTG
CAATGGCACAGGTTAAACTGGAACAGAGCGGTGCCGAAGTTGTTAAACCGGGTGCAA
GCGTTAAACTGAGCTGTAAAGCAAGCGGCTTTAACATCAAAGATAGCTATATGCATTGG
CTGCGTCAGGGTCCGGGTCAGCGTCTGGAATGGATTGGTTGGATTGATCCGGAAAATG
GTGATACCGAATATGCACCGAAATTTCAGGGTAAAGCAACCTTTACCACCGATACCAG
CGCAAATACCGCATATCTGGGTCTGAGCAGCCTGCGTCCGGAAGATACCGCAGTGTATT
 
ATTGTAATGAAGGCACCCCGACCGGTCCGTATTATTTCGATTATTGGGGTCAGGGCACC
CTGGTTACCGTTAGCAGCGGTGGTGGTGGTAGTGGTGGCGGTGGTTCAGGCGGTGGC
GGTAGCGAAAATGTTCTGACCCAGAGCCCGAGCAGCATGAGCGTTAGCGTTGGTGATC
GTGTTAATATTGCATGTAGCGCAAGCAGCAGCGTTCCGTACATGCACTGGCTGCAGCA
GAAACCGGGTAAAAGCCCGAAACTGCTGATTTATCTGACCAGCAATCTGGCAAGCGG
TGTTCCGAGCCGTTTTAGCGGTAGCGGTAGTGGCACCGATTATAGCCTGACCATTAGCA
GCGTGCAGCCTGAAGATGCAGCAACCTATTATTGTCAGCAGCGTAGCAGTTATCCGCT
GACCTTTGGTGGTGGCACCAAACTGGAAATTAAAGGGGGTGGTGGCTCAGGTGGCGG
AGGTGCAGAAGCAGGTATTACCGGTACATGGTCAGATCAGCTGGGTGATACCTTTATTG
TTACCGCAGGCGCAGATGGTGCACTGACCGGCACCTATGAAAATGCAGTTGGTGGTGC
AGAAAGCCGTTATGTGCTGACCGGTCGTTATGATAGCGCACCGGCAACCGATGGTAGC
GGCACCGCACTGGGTTGGACCGTTGCATGGAAAAATAACAGCAAAAATGCACATAGC
GCAACCACCTGGTCAGGTCAGTATGTGGGTGGTGCCGATGCCAAAATTAACACCCAGT
GGCTGCTGACCAGCGGTACAACCAATGCAAATGCCTGGAAAAGTACCCTGGTTGGTC
ATGATACATTCACCAAAGTTAAACCGAGCGCAGCAAGCCATCATCATCACCATCATTAA
SEQ ID NO:6 
MDKIAIVNMGSLFQQVAQKTGVSNTLENEFKGRASELQRMETDLQAKMKKLQSMKAGS
DRTKLEKDVMAQRQTFAQKAQAFEQDRARRSNEERGKLVTRIQTAVKSVANSQDIDLV
VDANAVAYNSSDVKDITADVLKQVK
SEQ ID NO:7
   GGGGSGGGG
SEQ ID NO: 8
   AAGGAGATATACATATGGATAAAATTGCCATTGTTAATAT
SEQ ID NO:9
   TTGAGATCTGCCATATGTTATTTCACTTGTTTCAGAACG
SEQ ID NO: 10
   AGAAGGAGATATACCATGAAATATCTGCTGCCGAC
SEQ ID NO:11
   CGCCGAGCTCGAATTTTAATGATGGTGATGATGATG
SEQ ID NO:12
   GGTATATCTCCTTCTTAAAGTTAAAC
SEQ ID NO: 13
   AATTCGAGCTCGGCGCGCCTGCAG

## Claims

1. A compound represented by the following formula (1) or formula (2): 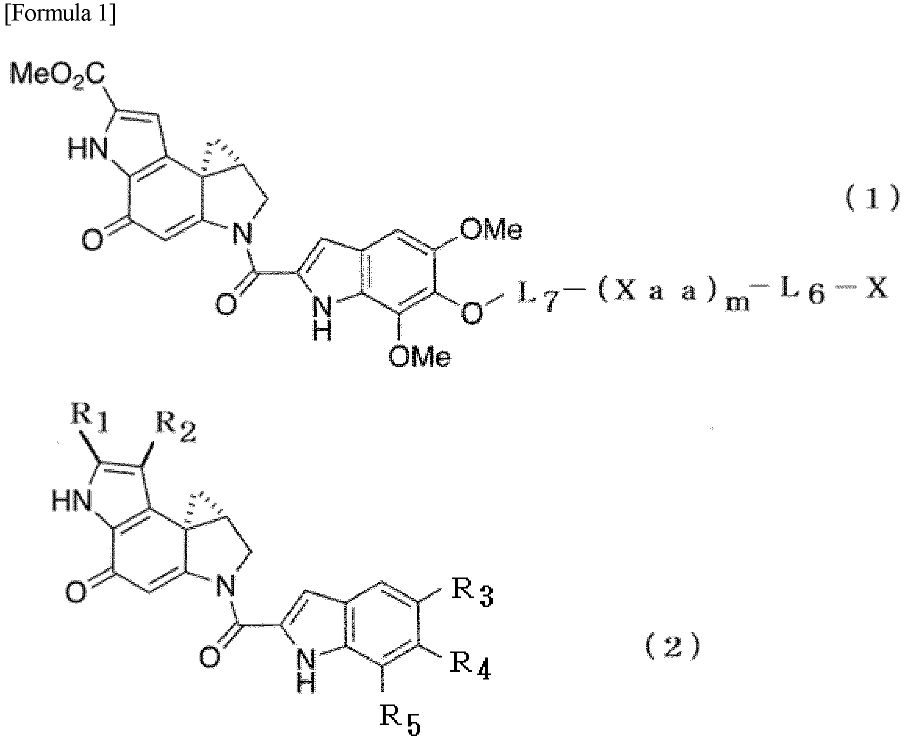 wherein R₁ and R₂ each independently represent a hydrogen atom, a lower alkyl group, or a lower alkoxycarbonyl group; one of R₃, R₄, and R₅ represents -O-L₇-(Xaa)ₘ-L₆-N₃, and the remaining two each independently represent a hydrogen atom, a lower alkyl group, or a lower alkoxycarbonyl group; X represents a reactive group; L₆ and L₇ each independently represent a divalent linking group; Xaa represents an amino acid residue; m represents an integer of 2 to 10; and Me represents a methyl group.

2. The compound according to claim 1, which is represented by the following formula (3A) or formula (3B): 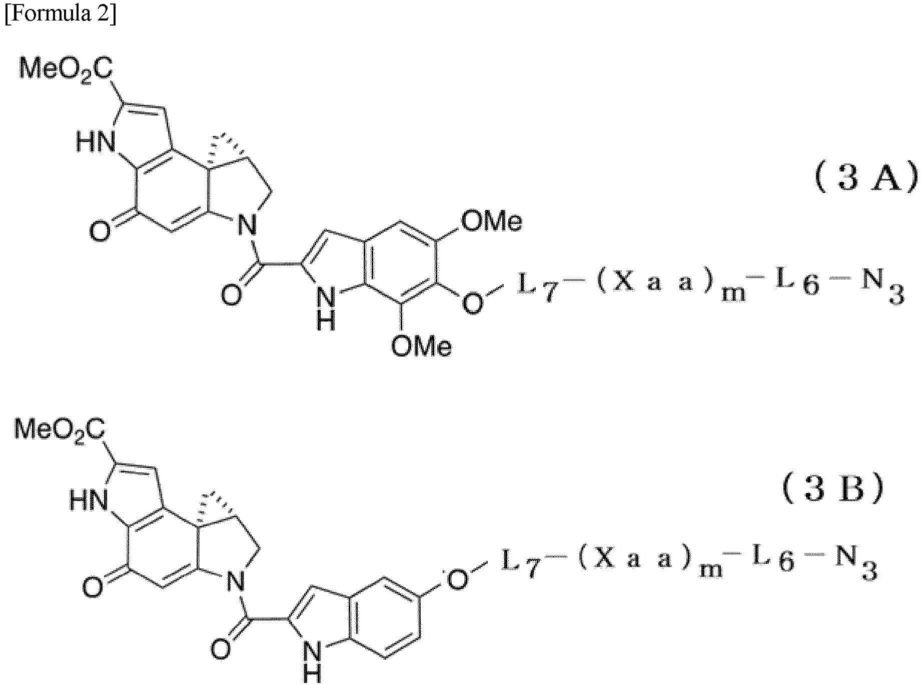 wherein the symbols are as defined in claim 1.

3. The compound according to claim 1 or 2, wherein -(Xaa)ₘ- is -Val-Ala-.

4. The compound according to any one of claims 1 to 3, wherein L₆ is -CO-(-O-CH₂)ₙ-(wherein n represents an integer of 1 to 10), and L₇ is -(CH₂)ₚ-NH- (wherein p represents an integer of 1 to 5).

5. A compound represented by the following formula (4): 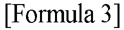 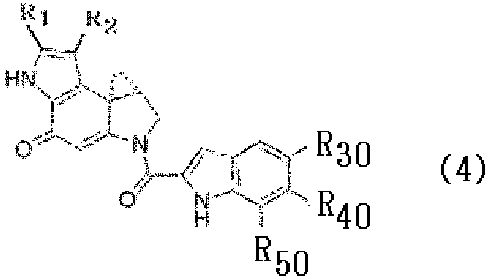 wherein
R₁ and R₂ each independently represent a hydrogen atom, a lower alkyl group, or a lower alkoxycarbonyl group; and
one of R₃₀, R₄₀ and R₅₀ represents the following: 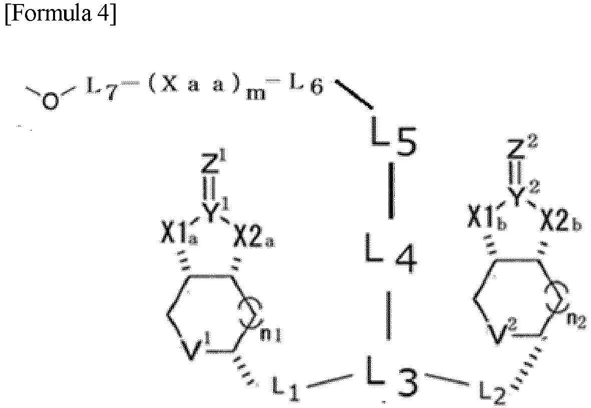
and the remaining two each independently represent a hydrogen atom, a lower alkyl group, or a lower alkoxyl group, wherein
X1a, X1b, X2a and X2b each independently represent O or NH;
Y¹ and Y² each independently represent C or S;
Z¹ and Z² each independently represent O, S or NH;
V¹ and V² each independently represent S or S⁺-O⁻, and n1 and n2 each independently represent an integer of 0 or 1;
L₁ and L₂ each independently represent a divalent linking group;
L₃ represents a trivalent linking group;
L₄, L₅, L₆ and L₇ each independently represent a divalent linking group; and
Xaa represents an amino acid residue, and m represents an integer of 2 to 10.

6. The compound according to claim 5, wherein
one of R₃₀, R₄₀ and is the following: 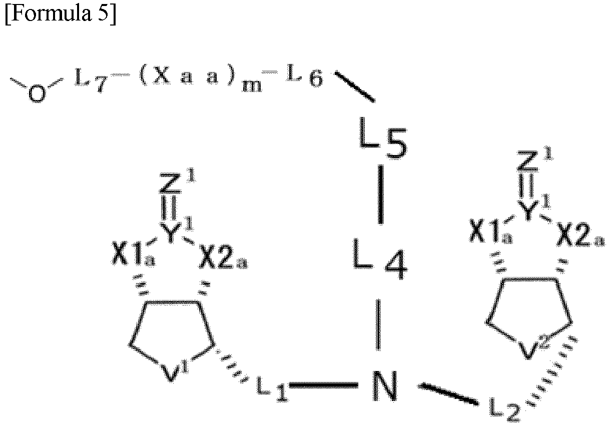 wherein individual symbols are as defined in claim 5.

7. A compound represented by the following formula (4A): 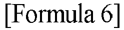 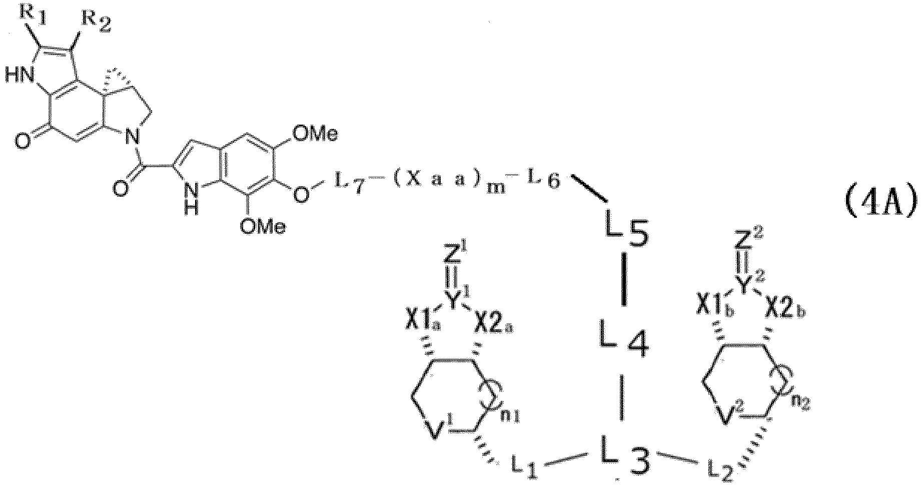 wherein
X1a, X1b, X2a and X2b each independently represent O or NH;
Y¹ and Y² each independently represent C or S;
Z¹ and Z² each independently represent O, S or NH;
V¹ and V² each independently represent S or S⁺-O⁻, and n1 and n2 each independently represent an integer of 0 or 1;
L₁ and L₂ each independently represent a divalent linking group;
L₃ represents a trivalent linking group;
L₄, L₅, L₆ and L₇ each independently represent a divalent linking group;
Xaa represents an amino acid residue, and m represents an integer of 2 to 10;
Me represents a methyl group; and
R₁ and R₂ each independently represent a hydrogen atom, a lower alkyl group, or a lower alkoxycarbonyl group.

8. The compound according to claim 5, which is represented by the following formula (5): 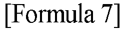 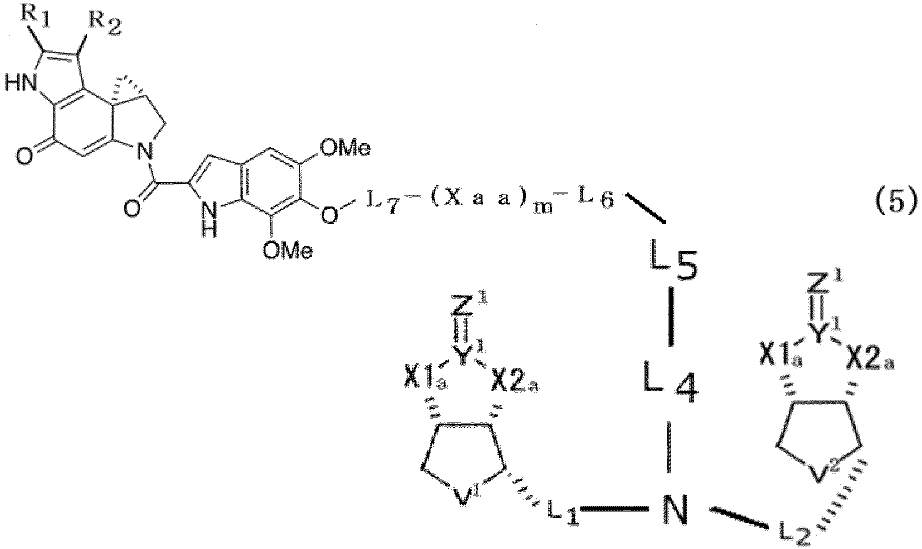 wherein individual symbols are as defined in claim 5.

9. The compound according to any one of claims 5 to 8, wherein X1a, X1b, X2a and X2b represent NH; Y¹ and Y² represent C; Z¹ and Z² represent NH; and V¹ and V² represent S.

10. The compound according to any one of claims 5 to 9, wherein L₁ and L₂ each independently represent a divalent group consisting of a combination of groups selected from -CONH-, -NHCO-, - COO-, -OCO-, -CO-, -O-, and an alkylene group containing 1 to 10 carbon atoms.

11. The compound according to any one of claims 5 to 10, wherein L₄ represents a group consisting of a combination of groups selected from -CONH-, -NHCO-, -COO-, -OCO-, -CO-, -O-, - NH-, and an alkylene group containing 1 to 10 carbon atoms.

12. The compound according to any one of claims 5 to 11, wherein L₅ represents a triazole group, -S-, -CONH-, -NHCO-, -COO-, -OCO-, -CO-, -O-, -NH-, an alkylene group containing 1 to 10 carbon atoms, or a group consisting of a combination of these groups.

13. The following compound or a salt thereof:

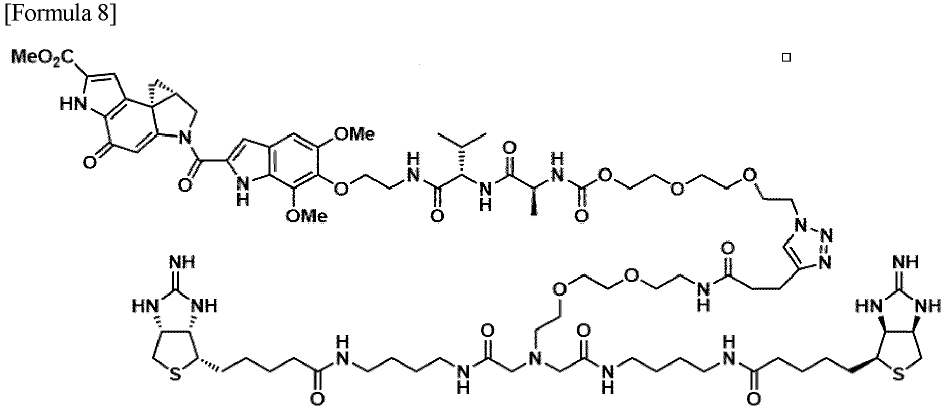

14. A therapeutic kit comprising:
(a) the compound according to any one of claims 5 to 13; and
(b) a conjugate of a mutant streptavidin comprising the amino acid sequence as set forth in SEQ ID NO: 1 (provided that a part of or the entire histidine tag at the C-terminus may be deleted) and a molecular probe.

15. The therapeutic kit according to claim 14, wherein the molecular probe is an anti-EREG antibody, an anti-CEA antibody, or an anti-HER2 antibody.
